# EUROPEAN PATENT APPLICATION

(11) **EP 2 441 776 A1**
(43) Date of publication of application: **18.04.2012**
(21) Application number: 10382270.6
(22) Date of filing: 15.10.2010
(51) Int. Cl.: C07K 14/78, C12N 15/62, C07K 19/00

(54) **Generation of multifunctional and multivalent polypeptide complexes with collagen XVIII trimerization domain**

(71) Applicant: Leadartis, S.L., 28008 Madrid (ES)
(72) Inventor: Álvarez Vallina, Luis, 28008 Madrid (ES); Cuesta Martínez, Ángel, 28008 Madrid (ES); Sainz Pastor, Noelia, 28008 Madrid (ES); Sanz Alcober, Laura, 28008 Madrid (ES)
(74) Representative: ABG Patentes, S.L.

(57) **Abstract**

The invention relates to the design of trimeric polypeptide complexes using polypeptide structural elements derived from the collagen XVIII protein, and their use in diagnostic and therapeutic systems *in vivo* and *in vitro.* The invention also relates to nucleic acids and vectors useful for producing said trimeric complexes.

SEQUENCE LISTING

<110>  Leadartis, S.L.

<120>  GENERATION OF MULTIFUNCTIONAL AND MULTIVALENT POLYPEPTIDE
       COMPLEXES WITH COLLAGEN XVIII TRIMERIZATION DOMAIN

<130>  P6424EP00

<160>  18

<170>  PatentIn version 3.5

<210>  1
<211>  59
<212>  PRT
<213>  Homo sapiens

<400>  1

Ser Gly Val Arg Leu Trp Ala Thr Arg Gln Ala Met Leu Gly Gln Val
1               5                   10                  15

His Glu Val Pro Glu Gly Trp Leu Ile Phe Val Ala Glu Gln Glu Glu
            20                  25                  30

Leu Tyr Val Arg Val Gln Asn Gly Phe Arg Lys Val Gln Leu Glu Ala
        35                  40                  45

Arg Thr Pro Leu Pro Arg Gly Thr Asp Asn Glu
    50                  55

<210>  2
<211>  177
<212>  DNA
<213>  Homo sapiens

<400>  2
tcaggggtga  ggctctgggc  tacacgccag  gccatgctgg  gccaggtgca  cgaggttccc
60

gagggctggc  tcatcttcgt  ggccgagcag  gaggagctct  acgtccgcgt  gcagaacggg
120

ttccggaagg  tccagctgga  ggcccggacg  ccactcccac  gagggacgga  caatgaa
177

<210>  3
<211>  15
<212>  PRT
<213>  Artificial Sequence

<220>
<223>  Flexible peptide linker

<400>  3

Gly Gly Gly Gly Ser Gly Gly Gly Gly Ser Gly Gly Gly Gly Ser
1               5                   10                  15


<210>  4
<211>  20
<212>  PRT
<213>  Artificial Sequence

<220>
<223>  Flexible peptide spacer

<400>  4

Gly Ser Ser Gly Ala Gly Gly Ser Gly Gly Ser Ser Gly Ser Asp Gly
1               5                   10                  15


Ala Ser Gly Arg
            20


<210>  5
<211>  14
<212>  PRT
<213>  Artificial Sequence

<220>
<223>  Flexible peptide spacer

<400>  5

Ser Gly Gly Thr Ser Gly Ser Thr Ser Gly Thr Gly Ser Thr
1               5                   10


<210>  6
<211>  15
<212>  PRT
<213>  Artificial Sequence

<220>
<223>  Flexible peptide spacer

<400>  6

Ala Gly Ser Ser Thr Gly Ser Ser Thr Gly Pro Gly Ser Thr Thr
1               5                   10                  15


<210>  7
<211>  7
<212>  PRT

<213>  Artificial Sequence

<220>
<223>  Flexible peptide spacer

<400>  7

Gly Gly Ser Gly Gly Ala Pro
1               5


<210>  8
<211>  8
<212>  PRT
<213>  Artificial Sequence

<220>
<223>  Flexible peptide spacer

<400>  8

Gly Gly Gly Val Glu Gly Gly Gly
1               5


<210>  9
<211>  4
<212>  PRT
<213>  Artificial Sequence

<220>
<223>  Flexible peptide linker

<400>  9

Gly Ser Pro Gly
1


<210>  10
<211>  13
<212>  PRT
<213>  Artificial Sequence

<220>
<223>  Flexible peptide linker

<400>  10

Ala Ala Ala Gly Gly Ser Gly Gly Ser Ser Gly Ser Arg
1               5                   10


<210>  11
<211>  8
<212>  PRT
<213>  Artificial Sequence

<220>
<223>  Flexible peptide linker

<400>  11

Gly Ser Gly Ser Gly Ser Gly Ser
1               5


<210>  12
<211>  21
<212>  PRT
<213>  Artificial Sequence

<220>
<223>  Flexible peptide linker

<400>  12

Ala Asn Ser Gly Ala Gly Gly Ser Gly Gly Ser Ser Gly Ser Asp Gly
1               5                   10                  15


Ala Ser Gly Ser Arg
            20


<210>  13
<211>  10
<212>  PRT
<213>  Artificial Sequence

<220>
<223>  Flexible peptide spacer

<400>  13

Pro Lys Pro Ser Thr Pro Pro Gly Ser Ser
1               5                   10


<210>  14
<211>  11
<212>  PRT
<213>  Artificial Sequence

<220>
<223>  Flexible peptide spacer

<400>  14

Ala Pro Ala Glu Thr Lys Ala Glu Pro Met Thr
1               5                   10


<210>  15
<211>  5
<212>  PRT

<213>  Artificial Sequence

<220>
<223>  Flexible peptide spacer

<400>  15

Ala Ala Ala Leu Glu
1               5


<210>  16
<211>  21
<212>  PRT
<213>  Artificial Sequence

<220>
<223>  Flexible peptide spacer

<400>  16

Ala Ala Ala Asn Ser Gly Ala Gly Gly Ser Gly Gly Ser Ser Gly Ser
1               5                   10                  15


Asp Gly Ala Ser Gly
            20


<210>  17
<211>  21
<212>  PRT
<213>  Artificial Sequence

<220>
<223>  Flexible peptide spacer

<400>  17

Gly Ser Ser Gly Ala Gly Gly Ser Gly Gly Ser Ser Gly Ser Asp Gly
1               5                   10                  15


Ala Ser Gly Arg Leu
            20


<210>  18
<211>  20
<212>  PRT
<213>  Artificial Sequence

<220>
<223>  Flexible peptide spacer

<400>  18

Asn Ser Gly Ala Gly Gly Ser Gly Gly Ser Ser Gly Ser Asp Gly Ala
1               5                   10                  15

Ser Gly Ser Arg
            20

## Description

### FIELD OF THE INVENTION

The invention relates generally to the design of trimeric polypeptide complexes using polypeptide trimerizing structural elements derived from the collagen XVIII protein and at least one heterologous moiety covalently linked to the C-terminal end of a monomer polypeptide comprising said trimerizing elements, and their use in diagnostic and therapeutic systems *in vivo* and *in vitro.* The invention also relates to nucleic acids and vectors useful for producing said trimeric polypeptide complexes.

### BACKGROUND OF THE INVENTION

Conversion of monovalent recombinant antibodies, such as scFv (single-chain variable fragments), Fabs (fragment antigen binding) or single-domains, into multivalent formats increases functional affinity, decreases dissociation rates when bound to cell-surface antigens, and enhances biodistribution [for a comprehensive review see Cuesta AM et al. Trends Biotech. 2010, 28:355-62].

The most common strategy to create multivalent antibodies has been the engineering of fusion proteins in which the antibody fragment makes a complex with homodimerization proteins. The most relevant recombinant dimeric antibodies are: the Fab₂ antibody and its single-chain counterpart, sc(Fab')₂ (Fab₂/sc(Fab)₂); the ZIP miniantibody; the scFv-Fc antibody; and the minibody. The miniantibody is a 64 kDa molecule generated by the association of two scFv fragments through dimerization domains. Leucine zippers (Fos or Jun) are employed to mediate dimerization of scFv in a miniantibody molecule. Fos-Fos and Jun-Jun homodimer formation allow efficient production of stable, secreted, monospecific miniantibodies, and Fos-Jun heterodimer formation allows production of bispecific miniantibodies. The scFv-Fc antibody is a small IgG-like recombinant antibody (100-105 kDa) generated by fusion of a scFv with human IgG₁ hinge and Fc regions (C_{H}2, and C_{H}3 domains). The 80 kDa minibody results from the fusion of a scFv to the human IgG₁ C_{H}3 domain. Other minibody-like molecules have been generated using the small immunoprotein technique, or by fusion to bacterial alkaline phosphatase.

Multimerization can be forced by shortening the peptide linker between the V domains of the scFv to self-associate into a dimer (diabody; 55 kDa). Further diabody derivatives are the triabody and the tetrabody, which fold into trimeric and tetrameric fragments by shortening the linker to less than 5 or to 0-2 residues, respectively. A different design for a (scFv)₂ is the 'bispecific T cell engager' (BITE). BITEs are bispecific single-chain antibodies consisting of two scFv antibody fragments, joined via a flexible linker, that are directed against a surface antigen on target cells and CD3 on T cells.

Oligomerization domains from non-Ig proteins have been used to make multivalent antibodies (i.e. molecular constructs based on the bacterial barnase-barstar module). The ribonuclease bamase (12 kDa) and its inhibitor barstar (10 kDa) form a very tight complex in which the N- and C-termini are accessible for fusion. This module, which has a dissociation constant of about 10⁻¹⁴ M, has been exploited to generate stable, trimeric scFv (about 130 kDa). Similar formats have been generated by fusion of a scFv to a cytokine that naturally exists in trimeric form, the tumor necrosis factor (TNF). TNFα genetically fused to the C-terminus of a scFv antibody forms a homotrimeric structure that retains both TNFα activity and the ability to bind antigen recognized by the scFv. Also, fusion proteins of scFv fragments with the C-terminal multimerization domain of the tumor-suppressor protein p53 or with a streptavidin subdomain results in tetrameric antibodies (scFv-SA)₄.

Another successful approach for the generation of multimeric antibodies has been the use of extracellular matrix-derived oligomerization domains. Trimerization of heterologous fusion proteins containing collagenous domain(s) has been accomplished by employing either a homogeneous or heterologous trimerization domain fused to the collagenous domain to drive the collagen triplex formation. Examples of a trimeroligomerizing domain include a C-propeptide of procollagens, a coiled-coil neck domain of collectin family proteins, a C-terminal portion of FasL and a bacteriophage T4 fibritin foldon domain [Frank et al., (2001) J Mol Biol 308: 1081-1089; Holler et al., (2003) Mol Cell Biol 23: 1428-1440; Hoppe et al., (1994) FEBS Lett 344: 191-195)]. A trimeric antibody, collabody (125 kDa), is based on the use of triplex-forming collagen-like peptides [(GPP)₁₀].

Fusion of the trimerization region of the C-terminal non-collagenous domain (NC1) of collagen XVIII to the C-terminus of a scFv confers a natural trimeric state to the fused antibody (trimerbody; 110 kDa). WO 2006/048252 discloses fusion proteins comprising the scFv fragment of an antibody and the NC1 domain of collagen XVIII, said domain comprising the trimerization domain and the homologous endostatin (ES) domain bound by a hinge peptide containing proteinase-sensitive sites. The trimeric constructs obtained are monospecific and trivalent.

However, for certain applications, it is convenient that molecules are multispecific (i.e., bi-, tri-specific, or even higher) and/or multivalent (i.e., tetra-, penta-, hexa-valent, or even higher). For full avidity of multivalent antibodies, all antigen-bonding sites need to be occupied. When targeting surface-bound molecules, orientation of binding domains in opposite directions, may facilitate simultaneous binding of all surface domains. Flexibility among antigen-binding sites is another important aspect in the design of multivalent antibodies required to cross-link surface receptors on either the same or adjacent cells. When a first interaction takes place, the possibility of establishing a second effective antigen-antibody interaction depends on the valence, spatial orientation and flexibility of the binding domain. Multiple binding events reduce the off-rates of the interaction, thereby increasing the retention time of the antibody bound to its target.

For tumor-targeting diagnostic or therapeutic applications, multispecific (i.e., bi-, tri-specific, or even higher) and/or multivalent (i.e., tetra-, penta-, hexa-valent, or even higher) antibodies will be most effective. In molecular trapping approaches, it is important to increase the antibody size above the renal threshold to retard clearance rates. Multispecific and/or multivalent antibodies, or mutivalent soluble truncated versions of membrane receptors (multiple trap) would have an increased binding stoichiometry to soluble cytokines and growth factors. Coupled with slowed clearance rates, the greater avidity of multivalent antibodies might translate soluble molecules. This can also be applied to other pathogenic particles against which antibody antidotes have been generated. These include toxins, such as botulinum neurotoxin A or anthrax toxin, and viruses, such as hepatitis viruses or varicella-zoster virus, in which multimerization has been demonstrated to be needed for effective neutralization.

Therefore, there is a need in the prior art to provide recombinant molecules containing a trimerization domain said recombinant molecules being multispecific and/or multivalent.

It has been now found that the trimerization region from collagen XVIII NC1 domain can be used to produce functionally active multispecific and/or multivalent C-terminal recombinant molecules thus opening the possibility of easily making multispecific, multivalent, recombinant molecules with different combinations of specificity and valency.

### BRIEF DESCRIPTION OF THE FIGURES

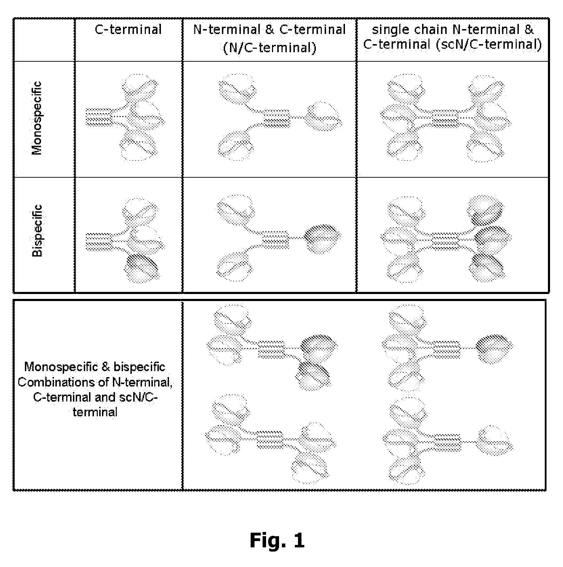

**Figure 1****.** Schematic representation of some illustrative, non-limitative, examples of trimeric polypeptide complexes (TPCs) comprising collagen XVIII trimerizing structural element (XVIIITSE) provided by the invention: mono- and bi-specific, trivalent C-terminal TPCs, mono- and bi-specific, trivalent N/C-terminal TPCs; and mono- and bi-specific, hexavalent single-chain N/C-terminal TPCs, and some combinations of N-terminal, C-terminal and/or single-chain N/C-terminal monospecific and bispecific TPCs.

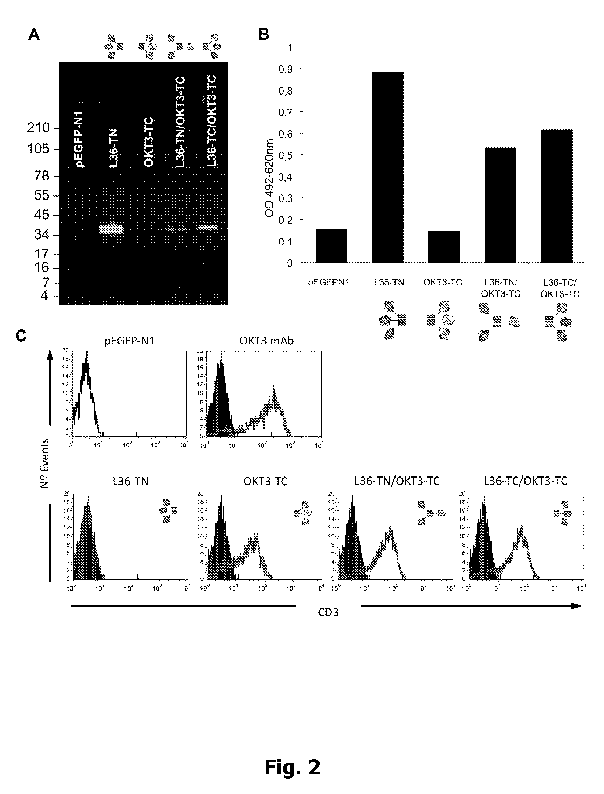

**Figure 2****.** Analysis of the presence of active N-terminal (L36-TN), C-terminal (OKT3-TC, L36-TC/OKT3-TC) or N-terminal and C-terminal (N/C) (L36-TN/OKT3-TC) trimeric polypeptide complexes (TPCs) in the supernatant of gene modified HEK-293T cells was demonstrated by Western Blotting (A), ELISA (B), and flow cytometry (C). (A) Neat supernatants were separated by SDS-PAGE in 4-20% Tris-Glycine gels and electro blotted. Proteins were detected using anti-myc mAb. Migration distances of molecular mass markers are indicated (kDa). (B) Analysis of the binding specificity of TPCs to laminin was demonstrated by ELISA against plastic immobilized laminin. Proteins were developed using an anti-myc mAb. Protein detection levels are not comparable due to differences in amounts of expressed protein. (C) Analysis by flow cytometry of the binding specificity of TPCs to CD3 expressed on the surface of human Jurkat T cells.

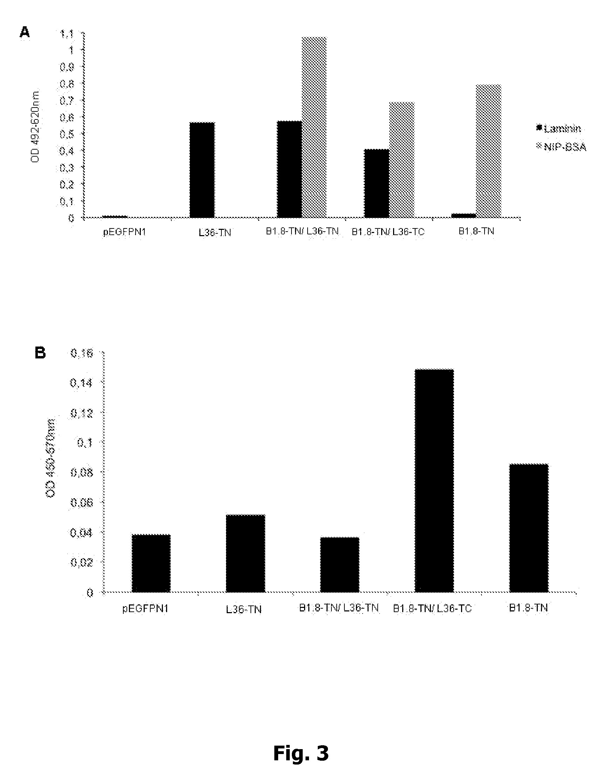

**Figure 3****.** Analysis of the bispecificity of N-terminal, and N/C-terminal trivalent trimeric polypeptide complexes (TPCs). (A) Binding activities of monospecific N-terminal (L36-TN, B1.8-TN), bispecific N-terminal (B1.8-TN/L36-TN) and bispecific N/C-terminal (B1.8-TN/L36-TC) TPCs were demonstrated by ELISA against plastic immobilized laminin and NIP-BSA conjugates. Bound proteins were detected with anti-myc mAb. (B) Bispecificity of bispecific N-terminal (B1.8-TN/L36-TN) and bispecific N/C-terminal (B1.8-TN/L36-TC) TPCs was demonstrated by ELISA against plastic immobilized laminin, and bound protein was then probed with NIP-BSA. Bound TPCs were developed using an anti-BSA antibody. Protein detection levels are not comparable due to differences in amounts of expressed protein.

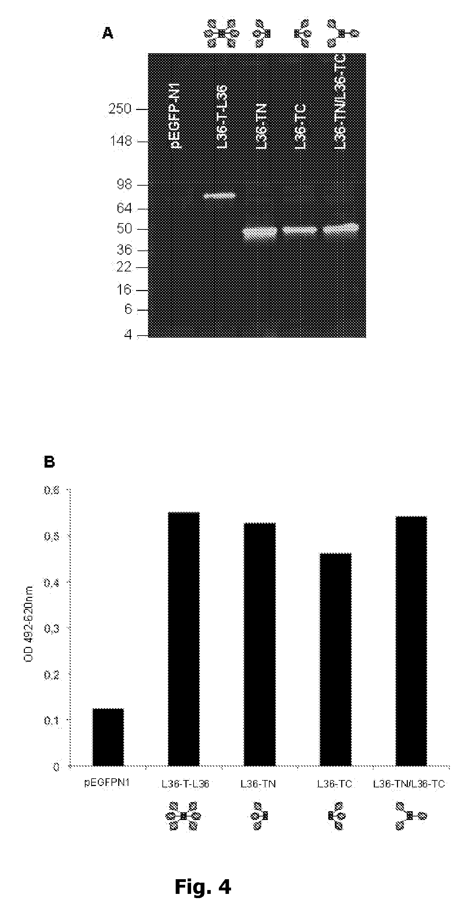

**Figure 4****.** Analysis of the presence of active monospecific N-terminal (L36-TN), C-terminal (L36-TC), N/C-terminal (L36-TN/L36-TC), and single chain N/C-terminal (L36-T-L36) trimeric polypeptide complexes (TPCs) in the supernatant of gene modified HEK-293T cells was demonstrated by Western Blotting (A), and ELISA (B). (A) Neat supernatants were separated by SDS-PAGE in 4-20% Tris-Glycine gels and electro blotted. Proteins were detected using anti-myc mAb. Migration distances of molecular mass markers are indicated (kDa). (B) Analysis of the binding specificity of TPCs to laminin was demonstrated by ELISA against plastic immobilized laminin.

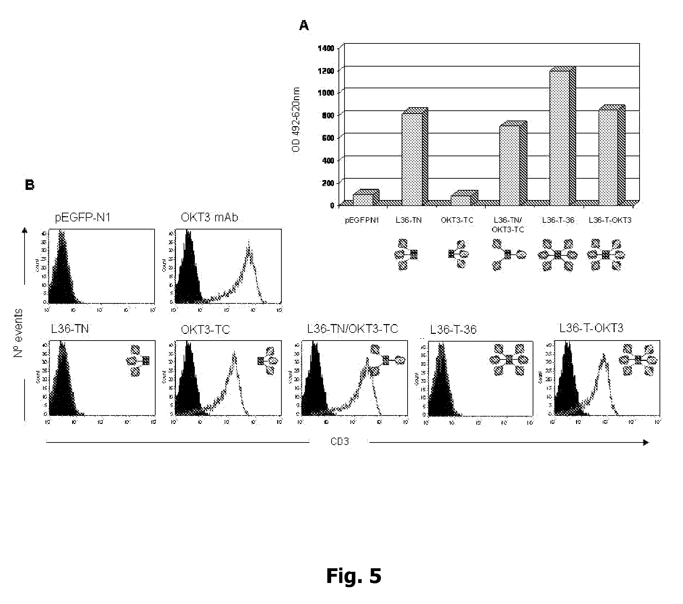

**Figure 5****.** Analysis of the presence of active monospecific N-terminal (L36-TN), C-terminal (OKT3-TC), and single chain N/C-terminal (L36-T-L36) trimeric polypeptide complexes (TPCs); or bispecific N/C-terminal (L36-TN/OKT3-TC), and single chain N/C-terminal (L36-T-OKT3) TPCs in the supernatant of gene modified HEK-293T cells was demonstrated by ELISA (A), and flow cytometry (B). (A) Analysis of the binding specificity of TPCs to laminin was demonstrated by ELISA against plastic immobilized laminin. (B) Analysis by flow cytometry of the binding specificity of TPCs to CD3 expressed on the surface of human Jurkat T cells.

### DETAILED DESCRIPTION OF THE INVENTION

In an aspect, the invention relates to a trimeric polypeptide complex, hereinafter referred to as the "trimeric polypeptide complex of the invention" or "TPC of the invention" (in plural "TPCs of the invention"), comprising three monomer polypeptides, wherein (i) each of said monomer polypeptides comprises a collagen XVIII trimerizing structural element (XVIIITSE) and (ii) at least one of said monomer polypeptides is covalently linked to at least one heterologous moiety, wherein said heterologous moiety is positioned C-terminally to said monomer polypeptide; i.e., the heterologous moiety is covalently linked to the C-terminal end of said monomer polypeptide.

As used herein, the term "collagen XVIII trimerizing structural element" or "XVIIITSE", refers to the portion of collagen XVIII which is responsible for trimerization between monomers of collagen XVIII. The term is also intended to embrace functionally equivalent variants of a XVIIITSE of a naturally occurring collagen XVIII, variants which have been modified in the amino acid sequence without adversely affecting, to any substantial degree, the trimerization properties relative to those of the native collagen XVIII molecule. Said modifications include, the conservative (or non-conservative) substitution of one or more amino acids for other amino acids, the insertion and/or the deletion of one or more amino acids, provided that the trimerization properties of the native collagen XVIII protein is substantially maintained, i.e., the variant maintains the ability (capacity) of forming trimers with other peptides having the same sequence at physiological conditions. The ability of a functionally equivalent variant to form trimers can be determined by conventional methods known by the skilled person in the art. For example, by way of a simple illustration, the ability of a functionally equivalent variant to form a trimer can be determined by using standard chromatographic techniques. Thus, the variant to be assessed is put under suitable trimerization conditions and the complex is subjected to a standard chromatographic assay under non denaturing conditions so that the eventually formed complex (trimer) is not altered. If the variant trimerizes properly, the molecular size of the complex would be three times heavier than the molecular size of a single molecule of the variant. The molecular size of the complex can be revealed by using standard methods such as analytical centrifugation, mass spectrometry, size-exclusion chromatography, sedimentation velocity, etc.

Although said XVIIITSE can derive virtually from any subject, in a particular embodiment said subject is a mammal, such as a mouse, a rat, a monkey, etc., preferably a human being. Thus, in a particular embodiment, the XVIIITSE is derived from human collagen XVIII. Specially preferred is a monomer polypeptide comprising at least one XVIIITSE derived from human collagen XVIII.

In a particular embodiment, said XVIIITSE consists of, or comprises, the N-terminal trimerization region of the collagen XVIII NC1 domain, such as the region comprising from residue 1207 to residue 1519 of human collagen XVIII, preferably from residue 1207 to residue 1266 [Takako S. et al. Structure, function and tissue forms of the C-terminal globular domain of collagen XVIII containing the angiogenesis inhibitor endostatin. The EMBO Journal Vol. 17 No. 1 pp.4249-4256, 1998; and Boudko, S.P., et al., Crystal Structure of Human Collagen XVIII Trimerization Domain: A Novel Collagen Trimerization Fold, J. Mol. Biol. (2009), doi:10.1016/ j.jmb.2009.07.057].

In another particular embodiment, the XVIIITSE is a polypeptide having the amino acid sequence shown in SEQ ID NO: 1 or a functionally equivalent variant thereof, i.e., a peptide derived from the peptide whose amino acid sequence is shown in SEQ ID NO: 1 by means of modification, insertion and/or deletion of one or more amino acids, provided that the trimerization property of the native collagen XVIII protein, namely from its NC1 domain, is substantially maintained, i.e., the functionally equivalent variant maintains the ability of the peptide whose amino acid sequence is shown in SEQ ID NO: 1 of forming trimers with other peptides having the same trimerization domain at physiological conditions. The ability of the functionally equivalent variant to form trimers can be determined by conventional methods as discussed above.

Illustrative, non-limitative, examples of functionally equivalent variants are those showing a degree of identity with respect to the peptide whose amino acid sequence is shown in SEQ ID NO: 1 equal to or greater than at least 30%, at least 40%, at least 50%, at least 60%, at least 70%, at least 80%, at least 90%, at least 95%, at least 96%, at least 97%, at least 98% or at least 99%. The degree of identity between two amino acid sequences can be determined by any conventional method, for example, by means of standard sequence alignment algorithms known in the state of the art, such as, for example BLAST [Altschul S.F. et al. Basic local alignment search tool. J Mol Biol. 1990 Oct 5; 215(3):403-10]. In a particular embodiment, the XVIIITSE is a functionally equivalent variant of the polypeptide having the amino acid sequence shown in SEQ ID NO: 1, said functionally equivalent variant having at least 80%, preferably at least 90%, more preferably at least 95% amino acid sequence identity with the sequence shown in SEQ ID NO: 1.

The person skilled in the art will understand that the amino acid sequences referred to in this description can be chemically modified, for example, by means of chemical modifications which are physiologically relevant, such as, phosphorylations, acetylations, etc.

It has been found that the XVIIITSE can be used to produce, among other trimeric polypeptide complexes (TPCs), functionally active mono- and bi-specific, trivalent C-terminal TPCs, mono- and bi-specific, trivalent N/C-terminal TPCs; and mono- and bi-specific, hexavalent single-chain N/C-terminal TPCs [please, see Figure 1, showing a schematic representation of said illustrative, non-limitative, examples of TPCs of the invention, and Example 1]. Additionally, it has been found that the XVIIITSE can be used to produce functionally active monospecific C-terminal TPCs with a single domain (V_{HH}) antibody as ligand binding domain or with a growth factor (e.g., VEGF). Therefore, the instant invention opens the possibility of easily making multi-specific (e.g., bi-, tri-, tetra-specific, etc.), multivalent (e.g., divalent, trivalent, tetravalent, pentavalent or hexavalent) recombinant molecules having different combinations of specificity and valency. By illustrative only, for tumor-targeting diagnostic or therapeutic applications, multispecific (i.e., bi-, tri-specific, or even higher) and/or multivalent (i.e., tetra-, penta-, hexa-valent, or even higher) antibodies will be most effective. In molecular trapping approaches, it is important to increase the antibody size above the renal threshold to retard clearance rates. Multispecific and/or multivalent antibodies, or mutivalent soluble truncated versions of membrane receptors (multiple trap) would have an increased binding stoichiometry to soluble cytokines and growth factors. Coupled with slowed clearance rates, the greater avidity of multivalent antibodies might translate soluble molecules. This can also be applied to other pathogenic particles against which antibody antidotes have been generated. These include toxins, such as botulinum neurotoxin A or anthrax toxin, and viruses, such as hepatitis viruses or varicella-zoster virus, in which multimerization has been demonstrated to be needed for effective neutralization.

By the term "heterologous moiety" is herein meant any chemical entity that can be fused or linked covalently to a XVIIITSE and to which the XVIIITSE is not natively covalently bound. Hence, the heterologous moiety can be any covalent partner moiety known in the art for providing desired binding, detection, or effector properties. Illustrative, non-limitative, examples of heterologous moieties include:
a) a ligand binding structure, such as a receptor molecule or the ligand binding part of a receptor molecule, such as for example a truncated form of the vascular endotelial factor receptor (VEGFR) 1 or 2; an antibody, an antigen binding antibody fragment, or a molecule having antibody characteristics, such as, for example, a single chain Fv (scFv), a diabody, a domain antibody (V_{H}), a nanobody (V_{HH}), binding molecules derived from the so-called alternative scaffolds, such as DARPins, anticalins, affibodies, adnectins, finomers, affilins, affitins, avimers, monobodies, miniantibodies, etc., or other ligand binding molecules such as avidin or streptavidin, or a lectin, etc.;
b) naturally occurring and/or engineered peptide sequences both in linear cyclic and forms, such as bicyclic peptide structures ('miniaturized' antibodies),
c) a toxin such as ricin, Pseudomonas exotoxin A, etc.;
d) a detectable label such as a fluorescence labelled molecule, a radioactively labelled molecule, or an enzymatically labelled molecule;
e) an *in situ* activatable substance, such as a molecule which can be induced by a magnetic field or by radiation to be radioactively or chemically active;
f) an enzyme such as a peroxidase, etc.;
g) a radioactive moiety such as a γ-, α-, β-, or β⁺-emitting molecule, e.g. a molecule comprising one or more radioactive isotopes selected from ¹⁴C, ³H, ³²P, ³³P, ²⁵S, ³⁸S, ³⁶Cl, ²²Na, ²⁴Na, ⁴⁰K, ⁴²K, ⁴³K, and any isotopes conventionally utilized for the purposes of facilitating detection of probes or the purposes of providing localized radiation so as to effect cell death;
h) a cytokine such as an interferon, an interleukin (e.g., interleukin-12, etc.), a leukotriene, an angiogenic growth factor such as vascular endotelial factor (VEGF), etc.;
i) an angiogenesis inhibitor such as endostatin, angiostatin, etc.
j) a Peptide Nucleic Acid (PNA);
k) a non-proteinaceous polymer such as a polymeric alkaloid;
l) a polyalcohol;
m) a polysaccharide;
n) a lipid;
o) a polyamine;
p) a photo cross-linking moiety, i.e., a chemical entity which effects cross-linking upon photo-activation;
q) a group facilitating conjugation of the monomer polypeptide to a target; or
r) a tag, i.e., a tag for the purpose of facilitating the isolation and purification of the TPC of the invention, for example an affinity purification tag such as a tag peptide; illustrative, non-limitative examples of said tags include polyhistidine [poly(His)] sequences, peptide sequences capable of being recognized by antibodies that may be used to purify the resultant fusion protein by immunoaffinity chromatography, for example epitopes derived from the hemagglutinin of the fever virus, c-myc tag, Strep tag, etc.

In a particular embodiment, said heterologous moiety is an antibody, in any format, for example a monoclonal antibody (mAb), or a recombinant fragment thereof, e.g., a single chain Fv fragment (scFv), a single domain antibody, etc. In a particular embodiment, said heterologous moiety is an antibody, in any format, which recognizes an angiogenic marker, for example, laminin, a component of the extracellular matrix (ECM), such as anti-laminin antibody L36 (Example 1) containing the variable heavy chain region (V_{H}) fused with a flexible linker, such as (Gly₄Ser)₃ [SEQ ID NO: 3] to the variable light chain (V_{L}) whose sequence has been previously described [Sanz L et al. Cancer Immunology and Immunotherapy, 2001 Dec; 50(10):557-65], wherein the N-terminus of said L36 scFv is linked to the C-terminus of said XVIIITSE, namely to the C-terminus of the human collagen XVIII NC1 trimerization region, through a flexible peptide spacer. Antibody L36 recognizes laminins of different animal species, for example, from mice, rats, humans, etc., since it interacts with a region that is very preserved among different animal species [Sanz L et al. EMBO J 2003, Vol. 22(7):1508-1517].

In another particular embodiment, said heterologous moiety is an antibody, in any format, against a marker present in a cell blood, e.g., a molecule expressed on the surface of a blood cell, for example, a CD antigen (i.e., any of a number of cell surface markers expressed by leukocytes and used to distinguish cell lineages, developmental stages, and functional subsets; such markers can be identified by monoclonal antibodies). In a particular embodiment, said blood cell is a lymphocyte (i.e., a type of white blood cell in the vertebrate immune system), such as a T cell or T lymphocyte. Illustrative, non-limitative examples of said antibodies include anti-CD3ε antibody OKT3, an antibody which recognizes CD3ε, a T lymphocyte marker, for example as an scFv (Example 1) containing the variable heavy chain region (V_{H}) fused with a flexible linker, namely (Gly₄Ser)₃ [SEQ ID NO: 3] to the variable light chain (V_{L}), wherein the N-terminus of said OKT3 scFv [Holliger P. et al. Carcinoembryonic Antigen (CEA)-specific T-Cell Activation in Colon Carcinoma Induced by Anti-CD3xAnti-CEA Bispecific Diabodies and B7xAnti-CEA Bispecific Fusion Proteins. Cancer Res. (1999) 59, 2909-16] is linked to the C-terminus of said XVIIITSE, namely to the N-terminus of the human collagen XVIII NC1 trimerization region, through a flexible peptide spacer [SEQ ID NO: 4] Other illustrative, non-limitative, examples of said antibodies include anti-CD4 antibodies (e.g., Clenoliximab, Keliximab, Zanolimumab, etc.), anti-CD11a antibodies (e.g., Efalizumab, etc.), anti-CD18 antibodies (e.g., Erlizumab, etc.), anti-CD20 antibodies (e.g., Afutuzumab, Ocrelizumab, Pascolizumab, etc.), anti-CD23 antibodies (e.g., Lumiliximab, etc.), anti-CD40 antibodies (e.g., Teneliximab, Toralizumab, etc.), anti-CD62L/L-selectin antibodies (e.g., Aselizumab, etc.), anti-CD80 antibodies (e.g., Galiximab, etc.), anti-CD147/Basigin antibodies (e.g., Gavilimomab, etc.), anti-CD154 antibodies (e.g., Ruplizumab, etc.), anti-BLyS (B-Lymphocyte Stimulator) antibodies (e.g., Belimumab, etc.), anti-CTLA-4 antibodies (e.g., Ipilimumab, Tremelimumab, etc.), anti-CAT antibodies (e.g., Bertilimumab, Lerdelimumab, Metelimumab, etc.), anti-Integrin antibodies (e.g., Natalizumab, etc.), anti-Interleukin-6 receptor antibodies (e.g., Tocilizumab, etc.), anti-LFA-1 antibodies (e.g., Odulimomab, etc.), anti- IL-2 receptor/CD25 antibodies (e.g., Basiliximab, Daclizumab, Inolimomab, etc.), etc.

As receptor agonists, the TPC of the invention, may be well-suited for receptors that trimerize upon ligand binding, such as the TNF family of receptors (CD134, CD40, FAS, CD27, CD30 or CD137). Members of the TNF family that lack death domains modulate T cell responses by providing costimulatory immune signals. Receptor crosslinking is essential for intracellular signaling [Watts, T.H. (2005) TNF/TNFR family members in costimulation of T cell responses. Annu. Rev. Immunol. 23, 23-68; and Zhou, Z. et al. (2008) Human glucocorticoid-induced TNF receptor ligand regulates its signaling activity through multiple oligomerization states. Proc. Natl. Acad. Sci. U. S. A 105, 5465-5470], and *in vitro* activation of these receptors requires oligomerization of their ligands [Wyzgol, A. et al. (2009) Trimer stabilization, oligomerization, and antibody-mediated cell surface immobilization improve the activity of soluble trimers of CD27L, CD40L, 41BBL, and glucocorticoid-induced TNF receptor ligand. J. Immunol. 183, 1851-1861]. One of such receptors is CD134, also known as OX40, whose engagement potently stimulates T cells and potentially inhibit Treg cells [Gough, M.J. et al. (2008) OX40 agonist therapy enhances CD8 infiltration and decreases immune suppression in the tumor. Cancer Res. 68, 5206-5215]. Other similar receptor/ligand systems are CD40 and CD137. In another particular embodiment, the heterologous moiety of the TPC of the invention is an antibody, in any format, against CD134, CD40, FAS, CD27, CD30 or CD137. Trimeric complexes can have superior efficiency as agonists of such receptors as they display conformations that allow simultaneous binding and engagement of three receptors, thereby eliciting activation and signaling of a larger subset of receptors.

In another particular embodiment, said heterologous moiety is an antibody, in any format, to a tumor-associated antigen (TAA), i.e., a new antigen acquired by a tumor cell line in the process of neoplastic transformation. Illustrative, non-limitative examples of said anti-TAA include anti-CEA (carcinoembryonic antigen) antibodies (e.g., antibody MFE-23, etc.), anti-TAG-72 (Tumor-associated glycoprotein 72) antibodies, anti-CD20 antibodies, anti-CD22 antibodies, anti-CD52 antibodies, anti-CD30 antibodies, anti-CD33 antibodies, anti-CD44v6 antibodies, anti-CD45 antibodies, anti-FBP (folate-binding protein) antibodies, anti-EGP-40 (epithelial glycoprotein 40) antibodies, anti-ganglioside (GD3) antibodies, anti-Lewis^{y} carbohydrate antigen antibodies, anti-platelet-derived growth factor (PDGFβR) antibodies, anti-cancer antigen 125 (CA-125) antibodies, anti-Mucin 1 antibodies, anti-cell surface associated (MUC1) or polymorphic epithelial mucin (PEM) antibodies, anti-B-lymphoma idiotype antibodies, anti-T-lymphoma idiotype antibodies, anti-HMW-MAA (high molecular weight-melanoma associated antigen) antibodies, anti-alphafetoprotein (AFP) antibodies, anti-renal cell carcinoma-selective antigen (e.g., antibody G250, etc.); antibodies against epidermal growth factor receptors (EGFR), such as EGFR (ErbB-1), HER2/c-neu (ErbB-2), Her 3 (ErbB-3) and Her 4 (ErbB-4); antibodies against EpCAM (Epithelial cell adhesion molecule), also been designated as TACSTD1 (tumor-associated calcium signal transducer 1); anti-CD326 antibodies; antibodies to chemokines; antibodies to chemokine receptors, etc.

In another particular embodiment, said heterologous moiety is an antibody, in any format, to an angiogenic marker, i.e., a protein associated with inappropriate or excessive angiogenesis, for example, extra domain-B containing fibronectin (EDB(+) FN), tenascin-c, laminin, etc.

In another particular embodiment, said heterologous moiety is an antibody, in any format, to a tumoral protein, i.e., a protein expressed within a tumoral context, for example, p32/gC1qR (Fogal V. et al. Mitochondrial/Cell-Surface Protein p32/gC1qR as a Molecular Target in Tumor Cells and Tumor Stroma. Cancer Res. 2008 68:7210), etc.

In another particular embodiment, said heterologous moiety is an antibody, in any format, to a pathogenic particle, for example a toxin, such as botulinum neurotoxin A, anthrax toxin, etc., a virus, such as a hepatitis virus, varicella-zoster virus, human immunodeficiency virus (HIV), etc., in which multimerization has been demonstrated to be needed for effective neutralization.

In another particular embodiment, said heterologous moiety is an antibody, in any format, to a growth factor, for example, vascular endothelial growth factor (VEGF), an angiogenic factor that stimulates the growth of new blood vessels. Illustrative, non-limitative examples of VEGF antibodies include bevacizumab [Kim KJ et al. Inhibition of vascular endothelial growth factor-induced angiogenesis suppresses tumour growth in vivo. Nature. 1993. 362:841-4], etc. Said antibody blocks VEGF biological activity. Additional illustrative, non-limitative, examples of said anti-growth factor antibodies include antibodies to tumor necrosis factor (TNF-α), antibodies to transforming growth factor (TGF), e.g., TGF-β1, antibodies to interferons (e.g., IFNγ, etc.), antibodies to interleukins (e.g., IL-12, IL-15, IL-4, IL-5, etc.), etc.

In another particular embodiment, said heterologous moiety comprises a non-Ig domain, such as a growth factor, for example, VEGF. Additional, non-limitative, examples of said growth factors include members of the epidermal growth factor family (EGF-family), members of the fibroblast growth factor (FGF) family, angiopoyetins, etc.

In another particular embodiment, the heterologous moiety comprises a specific affinity purification tag in order to obtain a substantially pure TPC of the invention, particularly if each one of the three monomer polypeptides comprises one affinity purification tag, said tags being different each other (e.g., affinity purification tags "a", "b" and "c", wherein tag "a" is recognized by binding substance A, tag "b" is recognized by binding substance B, and tag "c" is recognized by binding substance C), and it is subjected to a three-step affinity purification procedure designed to allow selective recovery of only such TPCs of the invention that exhibit affinity for the corresponding substances (A, B and C in the illustration). Said affinity purification tag can be fused directly in-line or, alternatively, fused to the monomer polypeptide via a cleavable linker, i.e., a peptide segment containing an amino acid sequence that is specifically cleavable by enzymatic or chemical means (i.e., a recognition/cleavage site). In a particular embodiment, said cleavable linker comprises an amino acid sequence which is cleavable by a protease such as an enterokinase, Arg-C endoprotease, Glu-C endoprotease, Lys-C endoprotease, factor Xa, etc.; alternatively, in another particular embodiment, said cleavable linker comprises an amino acid sequence which is cleavable by a chemical reagent, such as, for example, cyanogen bromide which cleaves methionine residues, or any other suitable chemical reagent. The cleavable linker is useful if subsequent removal of the affinity purification tags is desirable.

In a particular embodiment, when the heterologous moiety is a peptide, said heterologous moiety is preferably covalently linked to the XVIIITSE by via a peptide bond to the C-terminus of the XVIIITSE peptide chain. If, additionally, the TPC of the invention comprises a monomer polypeptide, said monomer polypeptide comprising a XVIIITSE and a heterologous moiety, said heterologous moiety being a peptide and being positioned N-terminally to said monomer polypeptide, then said heterologous moiety is preferably covalently linked to the XVIIITSE by via a peptide bond to the N-terminus of the XVIIITSE peptide chain.

In a particular embodiment, said heterologous moiety is directly covalently linked to the C-terminal end of said monomer polypeptide. However, in another particular embodiment, said heterologous moiety is not directly covalently linked to the C-terminal end of said monomer polypeptide but it is linked through a spacer, i.e., an inert connecting or linking peptide of suitable length and character, between the monomer polypeptide and the heterologous moiety [i.e., forming a "monomer polypeptide-spacer-heterologous moiety" structure]. In general, said spacer acts as a hinge region between said domains, allowing them to move independently from one another while maintaining the three-dimensional form of the individual domains. In this sense, a preferred spacer would be a hinge region characterized by a structural ductility or flexibility allowing this movement. The length of the spacer can vary; typically, the number of amino acids in the spacer is 100 or less amino acids, preferably 50 or less amino acids, more preferably 40 or less amino acids, still more preferably, 30 or less amino acids, or even more preferably 20 or less amino acids. Similarly, when the TPC of the invention comprises a monomer polypeptide in which a heterologous moiety is covalently linked to the N-terminal end of said monomer polypeptide, said heterologous moiety may directly covalently linked to the N-terminal end of said monomer polypeptide or, alternatively, said heterologous moiety can be linked to the N-terminal end of said monomer polypeptide through a spacer thus forming a "heterologous moiety-spacer-monomer polypeptide" structure.

Illustrative, non-limitative examples of spacers include a polyglycine linker; amino acid sequences such as those shown in SEQ ID NO: 5, SEQ ID NO: 6, SEQ ID NO: 7, SEQ ID NO: 8, etc. In a particular embodiment, said spacer is a peptide having structural flexibility (i.e., a flexible linking peptide or "flexible linker") and comprises 2 or more amino acids selected from the group consisting of glycine, serine, alanine and threonine. In another particular embodiment, the spacer is a peptide containing repeats of amino acid residues, particularly Gly and Ser, or any other suitable repeats of amino acid residues. Virtually any flexible linker can be used as spacer according to this invention. Illustrative examples of flexible linkers include amino acid sequences such as those shown in SEQ ID NO: 9, SEQ ID NO: 10, or SEQ ID NO: 11). Nevertheless, in a particular embodiment said spacer is a flexible linker comprising the amino acid sequence shown in SEQ ID NO: 12.

Alternatively, a suitable spacer can be based on the sequence of 10 amino acid residues of the upper hinge region of murine IgG3; said peptide (SEQ ID NO: 13) has been used for the production of dimerized antibodies by means of a coiled coil [Pack P. and Pluckthun, A., 1992, Biochemistry 31:1579-1584] and can be useful as a spacer peptide according to the present invention. Even more preferably, it can be a corresponding sequence of the upper hinge region of human IgG3 or other human Ig subclasses (IgG1, IgG2, IgG4, IgM and IgA). The sequences of human Igs are not expected to be immunogenic in human beings. Additional spacers that can be used in the instant invention include the peptides of the amino acid sequences GAP, AAA, or those shown in SEQ ID NO: 14 and SEQ ID NO: 15.

In a particular embodiment, the monomer polypeptide is covalently linked to only one heterologous moiety; in that case, said heterologous moiety is covalently linked by any of the above mentioned means, e.g., via a peptide bond, to the C-terminal end (C-terminus) of said monomer polypeptide comprising the XVIIITES peptide chain; this type of monomer polypeptide can be designated as "C-terminal monomer polypeptide" and represented as "XVIIITSE-HM", wherein HM means heterologous moiety and XVIIITSE is that previously defined.

In another particular embodiment, a monomer polypeptide is covalently linked to two heterologous moieties; in this embodiment, according to the invention, at least one of said heterologous moieties is covalently linked to the C-terminus of the monomer polypeptide comprising the XVIIITSE peptide chain. Thus, according to this particular embodiment, one of the heterologous moieties is linked to the C-terminus and the other one may linked to the N-terminus of said monomer polypeptide comprising the XVIIITES peptide chain, or, alternatively, both heterologous moieties may be linked each other and linked to the C-terminus of said monomer polypeptide comprising the XVIIITES peptide chain.

Thus, in a specific embodiment, a first heterologous moiety is linked to the C-terminus of said monomer polypeptide and a second heterologous moiety is linked to the N-terminus of said monomer polypeptide (this type of monomer polypeptide can be designated as N-/C-terminal monomer polypeptide and represented as "HM2-XVIIITSE-HM1", wherein HM1 means a first heterologous moiety, HM2 means a second heterologous moiety and XVIIITSE is that previously defined). Said first and second heterologous moieties can be equal or different. If the first and second heterologous moieties are equal the resultant monomer polypeptide will be monospecific (e.g., capable of reacting with only one antigenic determinant), whereas if the first and second heterologous moieties are different each other then the resultant monomer polypeptide will be bispecific (e.g., capable of reacting each heterologous moiety with one different antigenic determinant thus allowing the monomer polypeptide to react with two different antigenic determinants). Therefore, this particular embodiment, related to a monomer polypeptide comprising two heterologous moieties which are linked via peptide bonds to the C- and N-terminus, respectively, (i.e., N-/C-terminal monomer polypeptide) constitutes an interesting embodiment of the invention. This approach introduces a number of possibilities in terms of, for example, linking larger entities with oligomers of the invention by having specific activities coupled to each end of the monomers.

In another specific embodiment, a first heterologous moiety (HM1) is linked to a second heterologous moiety (HM2), in any order (HM1-HM2 or HM2-HM1), and also linked to only the C-terminus of the monomer polypeptide thus forming, e.g., an "in-line" or "tandem" structure, linked to the C-terminus, of the monomer polypeptide "XVTIIISE-HM1-HM2" or "XVIIITSE-HM2-HM1". Said first and second heterologous moieties can be equal or different. Preferably, in said "in-line" or "tandem" structures the heterologous moieties are linked each other by means of a spacer; in a particular embodiment, said spacer is flexible linking peptide or a "flexible linker" as defined above.

The skilled person in the art will understand that in further embodiments each monomer polypeptide of the trimeric polymeric complex (TPC) of the invention can be linked to one or more heterologous moieties, e.g., 2, 3, etc., at any of the ends (C-terminus and N-terminus) of said monomer polypeptide comprising the XVIIITES peptide chain, either at both ends or forming a tandem structure, provided that at least one of said heterologous moieties, in at least one of the monomer polypeptides which form the trtimeric complex of the invention, is covalently linked to the C-terminus of said monomer polypeptide comprising the XVIIITES peptide chain.

Thus, in a specific embodiment, at least one heterologous moiety is linked to the C-terminus of said monomer polypeptide and at least one heterologous moiety is linked to the N-terminus of said monomer polypeptide to render a monomer polypeptide; this type of monomer polypeptide can be represented as "(HM1)n-XVIIITES-(HM2)m", wherein the total number of heterologous moieties (n+m) is at least 3 and m is at least 1, each HM1 is, independently, equal or different to other HM1, and each HM2 is, independently, equal or different to other HM2. Further, each HM1 can be equal or different to each HM2. Illustrative, non-limitative, examples of this type of monomer polypeptide include monomer polypeptides in which one or more heterologous moieties are linked to the C-terminus of said monomer polypeptide and one or more heterologous moieties are linked to the N-terminus of said monomer polypeptide wherein the number of heterologous moieties is equal or higher than three.

As discussed above, the heterologous moieties can be directly linked each other, or, alternatively, in a particular embodiment, they can be linked though a spacer. The particulars or said spacer have been previously mentioned.

According to the instant invention, the TPC of the invention comprises three monomer polypeptides, wherein (i) each of said monomer polypeptides comprises a XVIIITSE, and (ii) at least one of said monomer polypeptides is covalently linked to at least one heterologous moiety, wherein said heterologous moiety is positioned C-terminally to said monomer polypeptide; i.e., the heterologous moiety is covalently linked to the C-terminal end of said monomer polypeptide.

Thus, in a particular embodiment, the TPC of the invention comprises three monomer polypeptides, wherein (i) each of said monomer polypeptides comprises a XVTSE and (ii) **only one** of said monomer polypeptides is covalently linked to at least one heterologous moiety, said heterologous moiety being positioned C-terminally to said monomer polypeptide. According to this particular embodiment, only one of the monomer polypeptides which constitute the TPC of the invention is covalently linked to at least one heterologous moiety, namely, to the C-terminal end of said monomer polypeptide which comprises the XVIIITSE peptide chain. In a particular embodiment, said monomer polypeptide is covalently linked to one heterologous moiety. In another particular embodiment, said monomer polypeptide is covalently linked to two or more, e.g., 2, 3, 4 or even more, heterologous moieties; said heterologous moieties can be equal or different each other. If the monomer polypeptide is covalently linked to two or more heterologous moieties, said heterologous moieties can be linked to the C-terminus, of the monomer polypeptide which comprises the XVIIITSE peptide chain, or alternatively, at least one heterologous moiety can be linked to the C-terminus of the monomer polypeptide and at least one heterologous moiety can be linked to the N-terminus of the monomer polypeptide.

Illustrative, non-limitative, examples of this embodiment of the TPC of the invention wherein only one monomer polypeptide is linked to at least one heterologous moiety, said heterologous moiety being positioned C-terminally to said monomer polypeptide, include TPCs of the invention in which:
- at least one heterologous moiety is linked to the C-terminus of the monomer polypeptide; or, alternatively,
- at least one heterologous moiety is linked to the C-terminus of the monomer polypeptide and at least one heterologous moiety is linked to the N-terminus of the monomer polypeptide.

In another particular embodiment, the TPC of the invention comprises three monomer polypeptides, wherein (i) each of said monomer polypeptides comprises a XVTSE and (ii) **each one of two** of said monomer polypeptides is covalently linked to at least one heterologous moiety, wherein at least one heterologous moiety is positioned C-terminally to at least one monomer polypeptide. According to this particular embodiment, each one of two of the three monomer polypeptides which constitute the TPC of the invention, independently, is covalently linked to at least one heterologous moiety with the proviso that in at least one of said monomer polypeptides which constitute the TPC of the invention the at least one heterologous moiety is covalently linked to the C-terminal end of said monomer polypeptide which comprises the XVIIITSE peptide chain. Thus, according to this particular embodiment, the TPC of the invention comprises a first monomer polypeptide covalently linked to at least one heterologous moiety and a second monomer polypeptide covalently linked to at least one heterologous moiety, subjected to the above mentioned proviso (i.e., in at least one of said monomer polypeptides, at least one heterologous moiety is covalently linked to the C-terminal end of said monomer polypeptide which comprises the XVIIITSE peptide chain), wherein the heterologous moieties linked to each of said first and second monomer polypeptide can be equal or different. Further, as discussed above, at least one of said heterologous moieties has to be linked to the C-terminus of the monomer polypeptide which comprises the XVIIITSE peptide chain, or alternatively, if any of the monomer polypeptides is covalently linked to two or more heterologous moieties, said heterologous moieties can be linked to the N-terminus, or to the C-terminus, of one of the monomer polypeptides which comprise the XVIIITSE peptide chain provided that in the other monomer polypeptide at least one heterologous moiety is linked to the C-terminus of the monomer polypeptide. The skilled person in the art will recognize that any combination of heterologous moieties can be present in this particular embodiment; i.e., in a specific embodiment, for example, a first monomer polypeptide can be linked to one or more heterologous moieties and a second monomer polypeptide can be linked to one or more heterologous moieties provided that at least one heterologous moiety is positioned C-terminally to at least one monomer polypeptide.

Illustrative, non-limitative, examples of this embodiment of the TPC of the invention wherein each one of only two monomer polypeptides are linked to at least one heterologous moiety, wherein at least one heterologous moiety is positioned C-terminally to said monomer polypeptide, include TPCs of the invention in which:
- at least one heterologous moiety is linked to the C-terminus of a first monomer polypeptide and at least one heterologous moiety is linked to the N-terminus of a second monomer polypeptide; or, alternatively,
- at least one heterologous moiety is linked to the C-terminus of a first monomer polypeptide and at least one heterologous moiety is linked to the C-terminus of a second monomer polypeptide; or, alternatively,
- at least one heterologous moiety is linked to the C-terminus of a first monomer polypeptide and at least one heterologous moiety is linked to the N-terminus of said first monomer polypeptide, and at least one heterologous moiety is linked to the N-terminus of a second monomer polypeptide; or, alternatively,
- at least one heterologous moiety is linked to the C-terminus of a first monomer polypeptide and at least one heterologous moiety is linked to the N-terminus of said first monomer polypeptide, and at least one heterologous moiety is linked to the C-terminus of a second monomer polypeptide; or, alternatively,
- at least one heterologous moiety is linked to the C-terminus of a first monomer polypeptide and at least one heterologous moiety is linked to the N-terminus of said first monomer polypeptide, and at least one heterologous moiety is linked to the N-terminus of a second monomer polypeptide and at least one heterologous moiety is linked to the C-terminus of said second monomer polypeptide.

In another particular embodiment, the TPC of the invention comprises three monomer polypeptides, wherein (i) each of said monomer polypeptides comprises a XVTSE and (ii) **each one of the three** monomer polypeptides is covalently linked to at least one heterologous moiety, wherein at least one heterologous moiety is positioned C-terminally to at least one monomer polypeptide. According to this particular embodiment, each one of the three monomer polypeptides which constitute the TPC of the invention, independently, is covalently linked to at least one heterologous moiety with the proviso that in at least one of said monomer polypeptides which constitute the TPC of the invention the at least one heterologous moiety is covalently linked to the C-terminal end of said monomer polypeptide which comprises the XVIIITSE peptide chain. Thus, according to this particular embodiment, the TPC of the invention comprises a first monomer polypeptide covalently linked to at least one heterologous moiety, a second monomer polypeptide covalently linked to at least one heterologous moiety, and a third monomer polypeptide covalently linked to at least one heterologous moiety, subjected to the above mentioned proviso (i.e., in at least one of said monomer polypeptides, at least one heterologous moiety is covalently linked to the C-terminal end of said monomer polypeptide which comprises the XVIIITSE peptide chain), wherein the heterologous moieties linked to each of said first, second and third monomer polypeptide can be equal or different each other, e.g., all the three heterologous moieties can be equal, or two of the three heterologous moieties can be equal each other and different to the other one, or, alternatively, all the three heterologous moieties can be different each other. The skilled person in the art will recognize that any combination of heterologous moieties can be present in this particular embodiment; i.e., in a specific embodiment, for example, a first monomer polypeptide can be linked to one or more heterologous moieties, the second monomer polypeptide can be linked to one or more heterologous moieties and the third monomer polypeptide can be linked to one or more heterologous moieties provided that at least one heterologous moiety is positioned C-terminally to at least one monomer polypeptide.

Illustrative, non-limitative, examples of this embodiment of the TPC of the invention wherein each one of the monomer polypeptides are linked to at least one heterologous moiety, wherein at least one heterologous moiety is positioned C-terminally to said monomer polypeptide, include TPCs of the invention in which:
- at least one heterologous moiety is linked to the C-terminus of a first monomer polypeptide, at least one heterologous moiety is linked to the N-terminus of a second monomer polypeptide and at least one heterologous moiety is linked to the N-terminus of a third monomer polypeptide; or, alternatively,
- at least one heterologous moiety is linked to the C-terminus of a first monomer polypeptide, at least one heterologous moiety is linked to the C-terminus of a second monomer polypeptide and at least one heterologous moiety is linked to the N-terminus of a third monomer polypeptide; or, alternatively,
- at least one heterologous moiety is linked to the C-terminus of a first monomer polypeptide, at least one heterologous moiety is linked to the C-terminus of a second monomer polypeptide and at least one heterologous moiety is linked to the C-terminus of a third monomer polypeptide; or, alternatively,
- at least one heterologous moiety is linked to the C-terminus of a first monomer polypeptide, at least one heterologous moiety is linked to the C-terminus of a second monomer polypeptide and at least one heterologous moiety is linked to the N-terminus (or, alternatively, at the C-terminus) of a third monomer polypeptide; or, alternatively,
- at least one heterologous moiety is linked to the C-terminus of a first monomer polypeptide, at least one heterologous moiety is linked to the N-terminus (or, alternatively, at the N-terminus) of a second monomer polypeptide and at least one heterologous moiety is linked to the C-terminus (or, alternatively, at the N-terminus) of a third monomer polypeptide; or, alternatively,
- at least one heterologous moiety is linked to the C-terminus of a first monomer polypeptide and at least one heterologous moiety is linked to the N-terminus of said first monomer polypeptide, at least one heterologous moiety is linked to the N-terminus of a second monomer polypeptide and at least one heterologous moiety is linked to the N-terminus of a third monomer polypeptide; or, alternatively,
- at least one heterologous moiety is linked to the C-terminus of a first monomer polypeptide and at least one heterologous moiety is linked to the N-terminus of said first monomer polypeptide, at least one heterologous moiety is linked to the C-terminus of a second monomer polypeptide and at least one heterologous moiety is linked to the C-terminus of a third monomer polypeptide; or, alternatively,
- at least one heterologous moiety is linked to the C-terminus of a first monomer polypeptide and at least one heterologous moiety is linked to the C-terminus of said first monomer polypeptide, at least one heterologous moiety is linked to the N-terminus of a second monomer polypeptide and at least one heterologous moiety is linked to the C-terminus of a third monomer polypeptide; or, alternatively,
- at least one heterologous moiety is linked to the C-terminus of a first monomer polypeptide and at least one heterologous moiety is linked to the N-terminus of said first monomer polypeptide, at least one heterologous moiety is linked to the N-terminus of a second monomer polypeptide and at least one heterologous moiety is linked to the C-terminus of said second monomer polypeptide and at least one heterologous moiety is linked to the N-terminus (or, alternatively, at the C-terminus) of a third monomer polypeptide; or, alternatively,
- at least one heterologous moiety is linked to the C-terminus of a first monomer polypeptide and at least one heterologous moiety is linked to the N-terminus of said first monomer polypeptide, at least one heterologous moiety is linked to the N-terminus of a second monomer polypeptide and at least one heterologous moiety is linked to the C-terminus of said second monomer polypeptide, and at least one heterologous moiety is linked to the N-terminus of a third monomer polypeptide and at least one heterologous moiety is linked to the C-terminus of said third monomer polypeptide.

Thus, as disclosed above, the TPC of the invention may be a homotrimer (i.e., all the monomer polypeptides are equal each other) or a heterotrimer (i.e., at least one of the monomer polypeptides is different from the other two monomer polypeptides).

Further, the specificity of the TPC of the invention can be designed in order to obtain mono- or multi-specific complexes. Thus, in a particular embodiment, when the heterologous moiety is an antibody or a fragment thereof, the TPC of the invention may be monospecific (e.g., when the heterologous moiety (e.g., antibody or fragment thereof) in each one of the three monomer polypeptides which constitute the TPC of the invention is the same, i.e., it is specific for just one antigen), bispecific (e.g., when one of the heterologous moieties (e.g., an antibody or fragment thereof recognizes a first antigen) in one of the monomer polypeptides which constitute the TPC of the invention is different from another heterologous moiety (e.g., an antibody or fragment thereof which recognizes a second antigen - wherein said second antigen is different from said first antigen) in the same or different monomer polypeptide), or even, the specificity of the TPC of the invention may be higher, i.e., the TPC of the invention can be trispecific (e.g., when one of the heterologous moieties (e.g., an antibody or fragment thereof recognizes a first antigen) in one of the monomer polypeptides which constitute the TPC of the invention is different from another heterologous moiety (e.g., an antibody or fragment thereof which recognizes a second (different) antigen) in the same or different monomer polypeptide) and different from another heterologous moiety (e.g., an antibody or fragment thereof which recognizes a third (different) antigen) in the same or different monomer polypeptide), or even higher (tetra-, penta-, or hexa-specific).

Additionally, the valence of the TPC of the invention can be designed in order to obtain mono- or multi-valent complexes. Thus, in a particular embodiment, when the heterologous moiety is an antibody or a fragment thereof, the TPC of the invention may be monovalent (i.e., the number of antigen binding sites possessed by the antibody or fragment thereof is one), bivalent (i.e., the number of antibodies or fragments thereof which are present in the TPC of the invention is two), trivalent (i.e., the number of antibodies or fragments thereof which are present in the TPC of the invention is three), tetravalent (i.e., the number of antibodies or fragments thereof which are present in the TPC of the invention is four), pentavalent (i.e., the number of antibodies or fragments thereof which are present in the TPC of the invention is five), or even hexavalent (i.e., the number of antibodies or fragments thereof which are present in the TPC of the invention is six). Multivalent formats of recombinant antibodies (e.g., scFv, Fabs, single-domains, etc.) increases functional affinity, decreases dissociation rates when bound to cell-surface antigens, and enhances biodistribution, among others.

Therefore, the TPC of the invention constitutes an enormously flexible platform for different applications, including protein engineering.

Effectively, a particularly interesting embodiment of the invention is the possibility of designing oriented molecular assemblies, where one or more functional entities are located C-terminally to the XVIIITSE and one or more functional entities are located N-terminally to said trimerizing element (XVIIITSE). Such types of design may be particularly advantageous where a certain relative ratio is desired among the different functional entities included in a specific molecular unit. Such type of design may in addition be used if one or more functional entities for either structural or functional reasons appear incompatible within the same construct. Such may be the case, e.g., if one or more of the functional entities are expressed by large or bulky protein domains which for steric reasons might prevent formation of the trimeric molecular unit due to sterical constraints.

Further, the possibility of constructing bi-polar three-way fusion proteins in which one functionality is placed at the C-terminus of the XVIIITSE and a different functionality is placed at the N-terminus of said XVIIITSE is additionally advantageous in applications where large spatial separation between the two functionalities are desirable for optimal function. Examples of such application are, e.g., the deployment of binding domains (e.g. antibody-derived binding modules) for recognition and binding to binding sites located at or close to large structures like cell membranes in cases where it is advantageous to allow for binding of the other end of the trimerized molecule to a different, but also bulky target.

Hence, as discussed above, the TPC of the invention may be used to join, e.g., bulky surfaces by said complex comprising at least one heterologous moiety which is positioned at the C-terminus of the XVIIITSE and at least one heterologous moiety which is positioned at the N-terminus of said XVIIITSE. The two heterologous moieties can be either part of the same monomer polypeptide or part of two separate monomer polypeptides.

The XVIIITSE will, essentially indiscriminately, form homo- and hetero-trimers with any molecule that also contains said trimerization module. For some applications, it may be advantageous to have available specially engineered derivatives of the XVIIITSE, which have been reengineered to disallow homo-trimer formation and hence only allow hetero-trimerization. Thus, an important embodiment of the monomer polypeptide which constitutes the TPC of the invention is constructed/reengineered so as to disfavour formation of complexes between identical XVIIITSEs; this also has the implication that said monomer polypeptides can advantageously be designed so as to disfavour formation of trimers including two monomer polypeptides having identical XVTSEs. One way of disfavouring the formation of homo-trimerization would be by "knobs into holes" mutagenesis.

The design/reengineering may be accomplished by introduction of amino acid substitution at sites in the monomer polypeptide intimately involved in the formation and stability of the trimer and, simultaneously, in a different construct introduce a compensatory amino acid substitution, all in all removing symmetry between individual monomer components of the triple helical structure so that the structural complementarity profile only allows the formation of hetero-trimers, but is incompatible with some or each of the homotrimer species.

The TPC of the invention may be prepared by methods generally known in the art, based, for example, techniques of recombinant protein production. Hence the invention also relates to a method of preparing the TPC of the invention, the method comprising isolating the TPC of the invention from a culture comprising a host cell which carries and expresses a nucleic acid fragment which encodes at least one of the monomer polypeptides of the TPC of the invention, and, optionally, subjecting the TPC of the invention to further processing.

The nucleic acid fragment which is mentioned above, hereinafter referred to as the nucleic acid of the invention, is also a part of the invention and is defined as a nucleic acid fragment in isolated form which encodes the polypeptide part of a monomer polypeptide according to the invention, said polypeptide part of said monomer polypeptide comprising the XVIIITSE and an heterologous moiety when the heterologous moiety is a peptide, said heterologous moiety being positioned C-terminally to said monomer polypeptide. In a particular, embodiment, the sequence of the nucleic acid encoding the XVIIITSE comprises the nucleotide sequence shown in SEQ ID NO: 2, which corresponds to the N-terminus region of the human collagen XVIII NC1 domain.

As mentioned above, the nucleic acid of the invention comprises the nucleotide sequence encoding the polypeptide part of a monomer polypeptide which is present in the TPC of the invention. The polypeptide part of said monomer polypeptide comprises the XVIIITSE and the heterologous moiety when the heterologous moiety is a peptide, said heterologous moiety being positioned C-terminally to said monomer polypeptide. As it has been previously mentioned, the monomer polypeptide may be fused to one or more heterologous moieties wherein at least one of said heterologous moieties is fused to the C-terminus of of the monomer polypeptide; thus, when said heterologous moieties are peptides, the nucleic acid of the invention comprises the nucleotide sequence encoding the XVIIITSE and the nucleotide sequence or sequences encoding said heterologous moiety or moieties. In that case, the 5' end of the nucleotide sequence encoding said heterologous moiety is fused to the 3' end of the nucleotide sequence encoding the XVIIITSE. Said nucleotide sequences can be operatively linked so that each sequence is correctly expressed by just one promoter or, alternatively, each nucleotide sequence is under the control of independent promoters. Said promoter(s) can be inducible or constitutive. The nucleic acid of the invention may include, if desired, operatively linked, the nucleotide sequence encoding the spacer between the monomer polypeptide and the heterologous moiety and/or, the nucleotide sequence encoding the cleavable linker between the monomer polypeptide and the heterologous moiety.

The nucleic acid of the invention can be prepared by traditional genetic engineering techniques.

The above mentioned host cell, hereinafter referred to as the host cell of the invention, which is also a part of the invention, can be prepared by traditional genetic engineering techniques which comprise inserting the nucleic acid of the invention into a suitable expression vector, transforming a suitable host cell with the vector, and culturing the host cell under conditions allowing expression of the polypeptide part of the monomer polypeptide which constitutes the TPC of the invention. Said vector comprising the nucleic acid of the invention, hereinafter referred to as the vector of the invention, is also a part of the invention. The nucleic acid of the invention may be placed under the control of a suitable promoter which may be inducible or a constitutive promoter. Depending on the expression system, the polypeptide may be recovered from the extracellular phase, the periplasm or from the cytoplasm of the host cell.

Suitable vector systems and host cells are well-known in the art as evidenced by the vast amount of literature and materials available to the skilled person. Since the present invention also relates to the use of the nucleic acid of the invention in the construction of vectors and in host cells, the following provides a general discussion relating to such use and the particular considerations in practising this aspect of the invention.

In general, of course, prokaryotes are preferred for the initial cloning of the nucleic acid of the invention and constructing the vector of the invention. For example, in addition to the particular strains mentioned in the more specific disclosure below, one may mention by way of example, strains such as *E. coli* K12 strain 294 (ATCC No. 31446), *E. coli* B, and *E. coli* X 1776 (ATCC No. 31537). These examples are, of course, intended to be illustrative rather than limiting.

Prokaryotes can be also utilized for expression, since efficient purification and protein refolding strategies are available. The aforementioned strains, as well as *E. coli* W3110 (F-, lambda-, prototrophic, ATCC No. 273325), bacilli such as *Bacillus subtilis,* or other enterobacteriaceae such as *Salmonella typhimurium* or *Serratia marcesans,* and various *Pseudomonas* species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. The pBR322 plasmid contains genes for ampicillin and tetracycline resistance and thus provides easy means for identifying transformed cells. The pBR322 plasmid, or other microbial plasmid or phage must also contain, or be modified to contain, promoters which can be used by the microorganism for expression.

Those promoters most commonly used in recombinant DNA construction include the B-lactamase (penicillinase) and lactose promoter systems and a tryptophan (trp) promoter system (EP 36776). While these are the most commonly used, other microbial promoters have been discovered and utilized, and details concerning their nucleotide sequences have been published, enabling a skilled worker to ligate them functionally with plasmid vectors. Certain genes from prokaryotes may be expressed efficiently in *E. coli* from their own promoter sequences, precluding the need for addition of another promoter by artificial means.

In addition to prokaryotes, eukaryotic microbes, such as yeast cultures may also be used. *Saccharomyces cerevisiase,* or common baker's yeast is the most commonly used among eukaryotic microorganisms, although a number of other strains are commonly available. For expression in *Saccharomyces,* the plasmid YRp7, for example, is commonly used. This plasmid already contains the *trpl* gene which provides a selection marker for a mutant strain of yeast lacking the ability to grow in tryptophan for example ATCC No. 44076 or PEP4-1 (Jones, 1977, Genetics, 85:23-33). The presence of the *trpl* lesion as a characteristic of the yeast host cell genome then provides an effective environment for detecting transformation by growth in the absence of tryptophan.

Suitable promoting sequences in yeast vectors include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination.

Other promoters, which have the additional advantage of transcription controlled by growth conditions are the promoter region for alcohol dehydrogenase-2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate in culture (tissue culture) has become a routine procedure in recent years. Examples of such useful host cell lines are VERO and HeLa cells, Chinese hamster ovary (CHO) cell lines, and W138, BHK, COS-7, Human Embryonic Kidney (HEK) 293 and MDCK cell lines.

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material; for example, commonly used promoters are derived from polyoma, Adenovirus 2, cytomegalovirus (CMV) and most frequently Simian Virus 40 (SV40). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *HindIII* site toward the *BglI* site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g., polyoma, adeno, etc.) or may be provided by the host cell chromosomal replication mechanism. If the vector is integrated into the host cell chromosome, the latter is often sufficient.

Upon production of the monomer polypeptide which constitutes the TPC of the invention, it may be necessary to process the polypeptides further, e.g. by introducing non-proteinaceous functions in the polypeptide, by subjecting the material to suitable refolding conditions (e.g. by using the generally applicable strategies suggested in WO 94/18227), or by cleaving off undesired peptide moieties of the monomer (e.g. expression enhancing peptide fragments which are undesired in the end product).

In the light of the above discussion, the methods for recombinantly producing said TPC of the invention or said monomer polypeptide which constitutes the TPC of the invention are also a part of the invention, as are the vectors carrying and/or being capable of replicating the nucleic acid of the invention in a host cell or in a cell-line. According to the invention the expression vector can be, e.g., a virus, a plasmid, a cosmid, a minichromosome, or a phage. Specially interesting are vectors which are integrated in the host cell/cell line genome after introduction in the host.

Another aspect of the invention are transformed cells (i.e., the host cell of the invention), useful in the above-described methods, carrying and capable of replicating the nucleic acid of the invention; the host cell can be a microorganism such as a bacterium, a yeast, or a protozoan, or a cell derived from a multicellular organism such as a fungus, an insect cell, a plant cell, or a mammalian cell. Specially interesting are cells from the bacterial species *Escherichia, Bacillus* and *Salmonella,* and a preferred bacterium is *E. coli.*

Yet another aspect of the invention relates to a stable cell line producing the monomer polypeptide which constitutes the TPC of the invention or the polypeptide part thereof, and preferably the cell line carries and expresses a nucleic acid of the invention. Specially interesting are cells derived from the mammalian cell lines HEK and CHO.

As it is evident for the skilled person in the art, the TPC of the invention may be a homotrimer (i.e., all the three monomer polypeptides are identical) or a heterotrimer (i.e., at least one of the three monomer polypeptides is different from the others).

When the TPC of the invention is a homotrimer, the method for recombinantly producing said homotrimer comprises inserting the nucleic acid of the invention into a suitable expression vector, transforming a suitable host cell with the vector, and culturing the host cell under conditions allowing expression of the monomer polypeptide according to the invention and trimerization thereof In this case, only one nucleic acid of the invention has to be prepared, said nucleic acid of the invention comprising the nucleotide sequence encoding the XVIIITSE and the nucleotide sequence encoding the polypeptide part of the heterologous moiety. The considerations applying to further processing mentioned above apply to this method also.

When the TPC of the invention is a heterotrimer, said heterotrimer may comprise (i) only one monomer polypeptide different from the other two monomer polypeptides, this two monomer polypeptides being identical each other, or, alternatively, (ii) three different monomer polypeptides.

In the first case, when the TPC of the invention is a heterotrimer wherein only one monomer polypeptide (MP1) is different from the other two monomer polypeptides (MP2), the method for recombinantly producing said heterotrimer comprises inserting a first nucleic acid of the invention comprising the nucleotide sequence encoding the XVIIITSE and the nucleotide sequence encoding said monomer polypeptide (MP1) into a suitable expression vector, and a second nucleic acid of the invention comprising the nucleotide sequence encoding the XVIIITSE and the nucleotide sequence encoding the other monomer polypeptide (MP2) into a suitable expression vector, transforming (cotransfecting) a suitable host cell with said vectors, and culturing the host cell under conditions allowing expression of the monomer polypeptides according to the invention and trimerization thereof to render the heterotrimer. In this case, two nucleic acids of the invention have to be prepared, one of them comprising the nucleotide sequence encoding a monomer polypeptide (e.g., MP1) and the other one encoding the other monomer polypeptide (MP2). The considerations applying to further processing mentioned above apply to this method also.

In the second case, when the TPC of the invention is a heterotrimer wherein all the three monomer polypeptides are different each other (e.g., MP1, MP2 and MP3), the method for recombinantly producing said heterotrimer comprises inserting a first nucleic acid of the invention comprising the nucleotide sequence encoding the XVIIITSE and the nucleotide the nucleotide sequence encoding monomer polypeptide 1 (MP1) into a suitable expression vector, inserting a second nucleic acid of the invention comprising the nucleotide sequence encoding the XVIIITSE and the nucleotide the nucleotide sequence encoding monomer polypeptide 2 (MP2) into a suitable expression vector, and inserting a third nucleic acid of the invention comprising the nucleotide sequence encoding the XVIIITSE and the nucleotide the nucleotide sequence encoding monomer polypeptide 3 (MP3) into a suitable expression vector, transforming (cotransfecting) a suitable host cell with said vectors, and culturing the host cell under conditions allowing expression of the monomer polypeptides according to the invention and trimerization thereof to render the heterotrimer. In this case, three nucleic acids of the invention have to be prepared, one of them comprising the nucleotide sequence encoding one monomer polypeptide (e.g., MP1), another encoding other monomer polypeptide (MP2), and another comprising the nucleotide sequence encoding another monomer polypeptide (MP3). The considerations applying to further processing mentioned above apply to this method also.

A very important aspect of the invention is the possibility of generating a system designed especially for the individual circumstances. The basic idea is that the artificial selection of heterologous moieties and optionally active components, and functional entities result in a unique system as will be further disclosed in the following.

Using the XVIIITSE as a vehicle for assembling monovalent scFv or Fab antibody fragments into oligomeric and multivalent entities offer design advantages also in terms of generating chimaeric artificial antibodies having desirable pharmacokinetic and pharmacodynamic properties. Small derivatives like monomeric scFv fragments or bivalent antibodies (e.g. diabodies, BITEs, and minibodies) are rapidly cleared from the circulatory system, whereas native Igs have longer half-life. Conversely, small derivatives like scFv and minibodies exhibit better extravasation properties. It is therefore expected that antibodies of a desired specificity may be optimized for particular diagnostic or therapeutic needs by engineering the pharmacological properties, using the XVIIITSE as a vehicle for controlled oligomerization of e.g. scFv fragments.

One example of such engineering would be the requirements for delivering a high dose of an imaging or toxin-conjugated antibody to a tumor, while ensuring as low a systemic exposure or imaging background as possible. In such case a XVIIITSE-fused or conjugated scFv fragment could be designed to exhibit strong multivalent binding to the tumor and rapid clearance of excess fusion protein or conjugate from circulation.

Accordingly, in a further aspect, the present invention also relates to the use of the TPC of the invention as a vehicle for assembled antibody fragments thus generating chimeric artificial antibodies having preselected pharmacokinetic and/or pharmacodynamic properties.

The use of specific delivery systems also play an important role in connection with the present invention in that such systems may be utilized with respect to different use of the present invention both with respect to the a more general therapeutic application and with respect to gene therapy. Examples of suitable drug delivery and targeting systems are disclosed in Nature 392 supp. (30 Apr. 1998).

Furthermore, a review of the imaging and therapeutic potential of a range of known antibody derivatives has been published [Holliger & Hudson, Nature Biotechnology, 2005, vol 23, 1126-1136; and Wu & Senter, Nature Biotechnology, 2005, vol 23, 1137-1146] In direct continuation of their conclusions it will be apparent that oligomerisation of antibody derivatives like scFv derivatives may extend current technology in the designer-antibody field in many important aspects, some of which will be elaborated below.

One of the well-known problems inherent to mouse monoclonal antibodies that have been "humanized" by grafting of the murine antigen combining site onto a human Ig framework is that antigenicity of the chimaeric product in human patients is often difficult to suppress entirely, resulting in sometimes life threatening immune reactions to the diagnostic or therapeutic humanized antibody product. The risks of such side-effects are expected to be much reduced if the designed antibody is assembled from purely human proteins or protein fragments. Since, in a particular embodiment, the XVIIITSE trimerization unit described herein is identical to a portion of human collagen XVIII that is already present in human plasma and tissue, there is good reason to expect that the XVIIITSE will not elicit an antigenic response in a human subject if it is introduced as a component of a chimaeric product that is not otherwise antigenic in humans.

Accordingly, in another aspect, the present invention relates to the use of a TPC of the invention or a monomer polypeptide according to the present invention as a component of a chimaeric product having low antigenicity in humans relative to formulations comprising on or more components of non-human origin.

In connection with the technology for radiolabelling of antibody derivatives, again, oligomerisation using XVIIITSE offer more elegant solutions to problems associated with labelling, as the XVIIITSE offers the possibility to construct one or two of the XVIIITSE monomer units in a heterotrimeric complex to harbour the site carrying the label. Thus, in this format labelling may also be confined to the non-antibody part of the complex, leaving the antigen-binding module entirely unmodified, and the complex may furthermore be formulated "in the field" as and when needed.

In molecular trapping approaches, it is important to increase the antibody size above the renal threshold to retard clearance rates. Trivalent antibodies (a particular embodiment of the TPC of the invention), or trivalent soluble truncated versions of membrane receptors (triple trap) would have an increased binding stoichiometry to soluble cytokines and growth factors. Coupled with slowed clearance rates, the greater avidity of trivalent antibodies might translate into a greater ability than their monovalent or bivalent counterparts to bind and sequester soluble molecules. This can also be applied to other pathogenic particles against which antibody antidotes have been generated. These include toxins, such as botulinum neurotoxin A or anthrax toxin, and viruses, such as hepatitis viruses or varicella-zoster virus, in which multimerization has been demonstrated to be needed for effective neutralization.

In many receptor-mediated signal transduction pathways signals are triggered by the clustering of receptor molecules on the cell membrane. The XVIIITSEs therefore have important applications in the study and exploitation of receptor signalling, especially for receptors that trimerize upon ligand binding such as those of the TNF family of receptors (eg. CD137, OX40) as ligands may be presented as trimers by genetic fusion or conjugation to a XVIIITSE unit. This also has important application in phage display technologies for discovering new ligands and new receptors as the engineering of a XVIIITSE unit fused in-line to a candidate ligand molecule will allow the display of a hetero-trimeric phage coat protein, in which only one of the monomer units is linked to the phage coat protein. This may be accomplished by appropriate insertion of amber codons at the site of fusion of phage coat protein to the XVIIITSE-ligand segment of the three-way fusion protein encoded by the recombinant phage. In appropriate *E*. *coli* cells the presence of this amber codon will result in translation termination in the majority of read-throughs, and hence most of the fusion protein product secreted to the periplasmic compartment in the phage-infected bacterium will be soluble XVIIITSE-ligand fusion protein, whereas a minority of the fusion protein will also contain a phage protein module. The majority of trimers that will be generated will therefore contain, at most, one monomeric unit that will ensure integration (display) in the mature recombinant phage particle.

A further advantage of the display technology described above relates to the fact that it is specially useful for selection on the basis of a relatively low affinity because of the entropic benefit contribution obtained by the proximity of the three binding moieties in confined spatial arrangement. Accordingly, the present invention in an important aspect, also relates to protein library technology wherein the XVIIITSE's described above are utilized.

The trimerization of candidate recombinant ligands is especially important as, for many receptors, the intracellular signal is induced by receptor clustering, which is only brought about if the external ligand exhibits multivalent binding to the receptor, so as to bridge two or more receptor molecules.

As mentioned above, it is an important aspect of the invention that the TPC of the invention or a monomer polypeptide according to the invention may be used as a component of a chimaeric product having low antigenicity in humans. As the monomer polypeptide can be of human origin it is believed that the antigenicity in humans is low relative to formulations comprising on or more components of non-human origin.

One primary use of a TPC of the invention or a monomer polypeptide according to the invention is for delivering an imaging or toxin-conjugated or genetically fused antibody to a target such as a tumor, or use as a vehicle delivering a substance to a target cell or tissue, as a vehicle for assembling antibody fragments into oligomeric or multivalent entities for generating chimeric artificial antibodies having pre-selected pharmacokinetic and/or pharmacodynamic properties.

The substance in question being one or more selected from the group of heterologous moieties as well as a pharmaceutical. Also a labelled construct wherein the label is coupled to one or more of the XVIIITSE monomer units is within the scope of the invention.

As explained in detail previously, an important and surprising use of the TPC of the invention or a monomer polypeptide according to the present invention is for protein library technology, such as phage display technology.

A further use according to invention includes the preparation and use of a pharmaceutical composition comprising the TPC of the invention or a monomer polypeptide according to the invention and optionally a pharmaceutically acceptable excipient. The composition may be administered by a route selected from the group consisting of the intravenous route, the intra-arterial route, the intravitreal route, the transmembraneus route of the buccal, anal, vaginal or conjunctival tissue, the intranasal route, the pulmonary route, the transdermal route, the intramuscular route, the subcutaneous route, the intratechal route, the oral route, inoculation into tissue such as a tumor, or by an implant.

It is obvious from the disclosure of the present invention that the treating or preventing of a disease may be a further aspect comprising administering to the subject in need thereof an effective amount of a pharmaceutical composition referred to above.

As used in the conventional pharmaceutical field the present invention includes a method wherein the TPC of the invention or a monomer polypeptide according to the invention is administered by a route selected from the group consisting of the intravenous route, the intra-arterial route, the transmembraneus route of the buccal, anal or vaginal tissue, intranasal route, intravitreal route, the pulmonary route, the transdermal route, intramuscular route, subcutaneous route, intratechal route, the oral route, inoculation into tissue such as a tumor, or by an implant.

Finally, the present invention is also related to the field of diagnosing as the skilled person would easily recognize that the XVIIITSE disclosed herein may also refer to a method for diagnosis comprising a TPC of the invention together with a diagnosing component coupled thereon.

The following example illustrate the invention and should not be considered as limitative of the scope thereof.

### EXAMPLE 1

### Generation and characterization of Functional C-Terminal, N-/C- Terminal, and N-/C-Terminal Single-Chain Trimeric Polypeptide Complexes (TPCs)

### 1. MATERIAL & METHODS

### Reagents and antibodies

The monoclonal antibodies (mAbs) used included 9E10 (Abcam, Cambridge, UK), specific to c-myc tag, OKT3 (Janssen-Cilag Pty Limited, USA), specific to human CD3ε, the PE-conjugated anti-human CD69 mAb (clone FN50, BD Biosciences, San José, CA, USA) and mouse anti-BSA mAb B2901 (Sigma-Aldrich).

The polyclonal antibodies, a rabbit anti-bovine serum albumin (BSA), a horseradish peroxidase (HRP)-conjugated goat anti-rabbit IgG, and an HRP-conjugated goat anti-mouse IgG (Fc specific) were from Sigma-Aldrich (St. Louis, MO, USA). The IRDye800 conjugated donkey anti-mouse IgG (H&L) was from Rockland Immunochemicals (Gilbertsville, PA, USA). Laminin extracted from the Engelbreth-Holm-Swarm (EHS) mouse tumor was from Invitrogen (Carlsbad, CA). BSA was conjugated with 4-hydroxy-5-iodo-3-nitrophenyl (NIP) (Sigma-Aldrich) in a molar ratio of 10:1 (NIP10-BSA) as previously described [Cuesta AM, et al. In vivo tumor targeting and imaging with engineered trivalent antibody fragments containing collagen-derived sequences. PLoS One. 2009;4(4):e5381].

### Cells and culture conditions

HEK-293T cells (human embryo kidney epithelia; CRL-11268) were cultured in Dulbecco's modified Eagle's medium (DMEM) supplemented with 10% (vol/vol) heat inactivated Fetal Calf Serum (FCS) (all from Invitrogen, Carlsbad, CA), unless otherwise stated. Jurkat clone E6-1 (TIB-152) cells were maintained in RPMI-1640 (Invitrogen) supplemented with heat-inactivated 10% FCS, referred as to RPMI complete medium (RCM). All of these cell lines were obtained from the American Type Culture Collection (Rockville, MD, USA).

### Construction of expression vectors

### N-terminal TPCs with human collagen XVIII trimerization domain

The DNA fragment [SEQ ID NO: 2] that encodes the trimerization region from the human collagen XVIII NC1 domain [SEQ ID NO: 1] was codon-optimized and synthesized by GeneArt AG (Regensburg, Germany), with an N-terminal GS linker [SEQ ID NO: 16] and C-terminal c-myc and hexahistidine tags.

This construct was subcloned through *Not*I/*Xba*I into the vector pCR3.1-L36 [Sanz L., et al. Single-chain antibody-based gene therapy: Inhibition of tumor growth by in situ production of phage-derived antibodies blocking functionally active sites of cell-associated matrices. Gene Ther (2002) 9: 1049-1053] to generate the vector **pCR3.1-L36-hNC1**, encoding the monomer polypeptide which constitutes the N-terminal TPC identified as **L36-TN** in this description, which comprises the scFv L36 containing the variable heavy chain region (V_{H}) fused with a (Gly₄Ser)₃ [SEQ ID NO: 3] linker to the variable light chain (V_{L}), wherein the C-terminus of said scFv L36 is linked to the N-terminus of the human collagen XVIII NC1 trimerization region through said flexible peptide linker [SEQ ID NO: 16]. N-terminal TPCs of other specificities, e.g., **B.18-TN**, were easily constructed by subcloning the corresponding scFv [Alvarez-vallina et al. Antigen-specific targeting of CD28-mediated T cell co-stimulation using chimeric single-chain antibody variable fragment CD28 receptors. Eur. J. Immunol. (1996) 26:2304-2309.] through *Cla*I/*Not*I [Cuesta AM, et al. In vivo tumor targeting and imaging with engineered trivalent antibody fragments containing collagen-derived sequences. PLoS One. 2009;4(4):e5381] in the the vector **pCR3.1-L36-hNC1.**

### C-terminal TPCs with human collagen XVIII trimerization domain

The DNA fragment coding for the trimerization region from the human collagen XVIII NC1 domain [SEQ ID NO: 1], a GS linker [SEQ ID NO: 4] and the anti-laminin scFv antibody L36 (V_{H}-V_{L}), was codon-optimized and synthesized by GeneArt AG. This construct was subcloned through *Cla*I/*Xho*I into the vector pCR3.1-L36 to generate the vector **pCR3.1-hNC1-L36,** encoding the monomer polypeptide which constitutes the C-terminal TPC identified as **L36-TC** in this description, which comprises the anti-laminin scFv antibody L36, wherein the N-terminus of said scFv antibody L36 is linked to the C-terminus of the human collagen XVIII NC1 trimerization region through said flexible peptide linker [SEQ ID NO: 4].

The DNA fragment [SEQ ID NO: 2] coding for the trimerization region from the human collagen XVIII NC1 domain [SEQ ID NO: 1], a GS linker [SEQ ID NO: 17] and the scFv of the anti-CD3 antibody OKT3 (V_{H}-V_{L}) was codon-optimized and synthesized by GeneArt AG. This construct was subcloned through *Bam*HI/*Xba*I into the vector pCR3.1-hNC1-L36 to generate the vector **pCR3.1-hNC1-OKT3**, encoding the monomer polypeptide which constitutes the C-terminal TPC identified in this description as **OKT3-TC**, which comprises the scFv of the anti-CD3 antibody OKT3, wherein the N-terminus of said scFv OKT3 is linked to the C-terminus of the human collagen XVIII NC1 trimerization region, through said flexible peptide linker [SEQ ID NO: 17].

### Single-chain N/C-terminal TPCs with human collagen XVIII trimerization domain

The monospecific single-chain N/C-terminal TPC of L36 was generated by cloning the DNA fragment encoding the flexible linker [SEQ ID NO: 4] and the anti-laminin scFv antibody L36 of pCR3.1-hNC1-L36 into the vector pCR3.1-L36-hNC1, through *Bam*HI/*Xba*I, in order to generate the vector **pCR3.1-L36-hNC1-L36,** encoding the monomer polypeptide which constitutes the single-chain N/C-terminal TPC identified as **L36-T-L36** in this description, which comprises (i) the scFv of the antibody L36, wherein the C-terminus of said scFv is linked to the N-terminus of the human collagen XVIII NC1 trimerization region [SEQ ID NO: 1], through a flexible peptide linker [SEQ ID NO: 16], and (ii) the scFv of the antibody L36 wherein the N-terminus of said antibody L36 is linked to the C-terminus of the human collagen XVIII NC1 trimerization region through said flexible peptide linker [SEQ ID NO: 4].

The bispecific single chain N/C-terminal TPC of L36 and OKT3 was generated by cloning the DNA fragment encoding the flexible linker [SEQ ID NO: 4] and the anti-human CD3 scFv antibody OKT3 into the vector pCR3.1-L36-hNC1, through *Bam*HI/*Xba*I, in order to generate the vector pCR3.1-L36-hNC1-21-OKT3, encoding the monomer polypeptide which constitutes the single-chain N/C-terminal TPC identified as L36-T-OKT3 in this description, which comprises (i) the scFv of the antibody L36, wherein the C-terminus of said scFv is linked to the N-terminus of the human collagen XVIII NC1 trimerization region, through a flexible peptide linker [SEQ ID NO: 16], and (ii) the scFv of the antibody OKT3 wherein the N-terminus of said antibody OKT3 is linked to the C-terminus of the human collagen XVIII NC1 trimerization region through said flexible peptide linker [SEQ ID NO: 4].

### Cell transfections and expression of recombinant antibodies

HEK-293T cells were transfected with the appropriate expression vectors, using Superfect (QIAGEN GmbH, Hilden, Germany). Supernatants from transiently transfected cell populations were collected after 48-72 h and analyzed for protein expression by ELISA and Western blotting using anti-myc mAb 9E10.

### ELISA

Neat supernatants were incubated in microtiter plates (Nunc, Denmark), previously coated with 10 [µg/ml laminin and blocked with 5% milk/PBS. Bound proteins were detected with mouse anti-myc mAb 9E10. When testing bispecificity, proteins were detected using BSA conjugated with NIP, and mouse anti-BSA mAb B2901 (Sigma-Aldrich).

### Western Blotting

Neat supernatant were separated by reducing SDS-PAGE in 4-20% Tris-Glycine gels (Bio-Rad Laboratories Ltd., Hemel Hampstead, UK) and transferred onto nitrocellulose membranes. After blocking with LI-COR blocking solution (LI-COR, Lincoln, NE, USA), proteins were detected with mouse anti-myc mAb 9E10 and donkey anti-mouse IgG (H&L) IRDye800 conjugated. Images were taken using an Odyssey Infrared Imaging system (LI-COR).

### Flow Cytometry

The ability of the different TPCs containing the OKT3-derived scFv to recognize its cognate antigen (CD3ε) on cells was tested by labelling Jurkat cells with protein samples, and detecting with mouse anti-myc 9E10 mAb and anti-mouse polyclonal antibody conjugated to phycoerythrin (PE). All samples were analyzed using an EPICS XL flow cytometer (Beckman Coulter).

The ability of the different TPCs to cross-link and activate Jurkat cells was carried out in solution and in solid phase. For activation in solution, Jurkat cells were mixed with monospecific or bispecific TPCs and incubated overnight. Cell activation was estimated by detecting the expression of CD69 on the cell surface by flow cytometry, using PE-conjugated anti-human CD69 mAb (FN50). For activation in solid phase, bispecific TPCs were added to wells with immobilized laminin and washed. Jurkat cells were incubated overnight and processed as above.

### II. RESULTS

### Design and expression of TPC constructs

Inventors have previously shown that TPCs of different specificities, including the anti-laminin L36 scFv, containing the C-terminal trimerization domain of murine collagen XVIII NC1 were produced in HEK-293 cells in a homotrimeric, functionally active form [Sánchez-Arevalo Lobo (2006). Int J Cancer. 119:455-62.]

In the present study, the inventors have extended the scope of TPC formats by designing constructs with C-terminal scFvs. The anti-laminin and anti-CD3 scFvs, L36 and OKT3 respectively, were C-terminally fused to the trimerization region from the human collagen XVIII NC1 domain. The trimeric polypeptide complexes (L36-TC and OKT3-TC) were secreted as functional proteins in HEK-293T cells transfected with plasmid encoding C-terminal TPCs, N-terminal TPCs or co-transfected with plasmids encoding C-terminal TPCs with different specificity, N-terminal TPCs with different specificity or both C-terminal TPCs and N-terminal TPCs plasmids. Transfection efficiency was determined by transfecting HEK-293T cells with the pEGFP-N1 plasmid (Clontech) coding the green-fluoresecence protein (GFP). Western blotting analysis revealed bands of about 38-40 kDa (Fig. 2A and 4A), the expected size of C-terminal TPCs or N-terminal TPCs monomer polypeptides in denaturing SDS-PAGE.

### Antigen binding activity

The ability of monospecific or bispecific N-terminal or C-terminal TPCs, monospecific or bispecific N/C-terminal TPCS, and monospecific or bispecific single chain N/C-terminal TPCs, to recognize their cognate antigens was demonstrated by ELISA (L36-derived constructs) and by flow cytometry (OKT3-derived constructs). An ELISA on immobilized laminin (Fig. 2B, 3A, 4B and 5A) showed that only samples containing L36 were able to bind, regardless of their N- or C-terminal position. In flow cytometry assay, Jurkat cells were probed with neat supernatants and detected with anti-myc mAb 9E10, results revealed that only the constructions containing OKT3 were able to detect CD3ε on the cell surface (Fig. 2C and 5B). The OKT3 in native format (mAb) was used as positive control.

The bispecificity of N-terminal or C-terminal TPCs, N/C-terminal TPCS, and single chain N/C-terminal TPCs was studied by sandwich ELISA as previously described [Cuesta AM, et al. In vivo tumor targeting and imaging with engineered trivalent antibody fragments containing collagen-derived sequences. PLoS One. 2009;4(4):e5381]. Briefly, conditioned medium from double-transfected HEK-293T was added to laminin-coated wells and, after washing, the laminin-bound TPCs were able to capture soluble NIP₁₀-BSA (Fig. 3B). TPCs in the supernatant from single-transfected HEK-293T cells were shown to bind to laminin, but the bound TPC did not capture, in turn, NIP₁₀-BSA (Fig. 3B).

The monospecific anti-laminin single-chain TPC (L36.T-L36) and the bispecific anti-laminin and anti-CD3 single-chain TPC (L36-T-OKT3) were secreted as functional proteins in HEK-293T cells, as efficiently as N-terminal, C-terminal, and N/C anti-laminin TPCs (Fig. 4 and 5). Western blotting analysis revealed a band of 65-70 kDa (Fig. 4A), the expected size of a single chain TPC monomer in denaturing SDS-PAGE. Although the amount of L36-T-L36 expressed was slightly lower than control TPCs, its ability to recognize plastic immobilized laminin seemed to be higher by ELISA (Fig. 3), and it gives an indication of its hexavalency. Bispecific anti-laminin and anti-CD3 single-chain (L36-T-OKT3) TPCs recognized both plastic immobilized laminin (Fig. 5A) and CD3ε on the cell surface of human Jurkat T cells (Fig. 5B).

### III. CONCLUSIONS

It has been demonstrated the possibility of producing functionally active:
1. Mono and bispecific C-terminal TPCs.
2. Mono and bispecific N-/C-terminal TPCs.
3. Mono and bispecific, hexavalent single-chain TPCs. This opens the possibility of easily making bispecific (or even more, e.g., trispecific, tetraspecific, etc.), multivalent (e.g., divalent, trivalent, tetravalent, pentavalent or hexavalent) TPCs and several other combinations.

## Claims

1. A trimeric polypeptide complex comprising three monomer polypeptides, wherein (i) each of said monomer polypeptides comprises a collagen XVIII trimerizing structural element (XVIIITSE) and (ii) at least one of said monomer polypeptides is covalently linked to at least one heterologous moiety, wherein said heterologous moiety is positioned C-terminally to said monomer polypeptide.

2. Trimeric polypeptide complex according to claim 1, wherein said XVIIITSE consists of, or comprises, the N-terminal trimerization region of the collagen XVIII NC1 domain, preferably, a polypeptide having the amino acid sequence shown in SEQ ID NO: 1 or a functionally equivalent variant thereof.

3. Trimeric polypeptide complex according to claim 1, wherein said heterologous moiety is an antibody or a recombinant fragment thereof, or a detectable label.

4. Trimeric polypeptide complex according to claim 1, wherein said monomer polypeptide is linked to said heterologous moiety through a spacer.

5. Trimeric polypeptide complex according to claim 1, wherein said monomer polypeptide is covalently linked to two or more heterologous moieties; wherein said two or more heterologous moieties are, independently, equal or different each other.

6. Trimeric polypeptide complex according to claim 5, wherein
at least one heterologous moiety is positioned C-terminally to said monomer polypeptide and at least one heterologous moiety is positioned N-terminally to said monomer polypeptide, or alternatively, wherein
said two or more heterologous moieties are positioned C-terminally to said monomer polypeptide.

7. Trimeric polypeptide complex according to claim 1, which comprises a first monomer polypeptide covalently linked to at least one heterologous moiety and a second monomer polypeptide covalently linked to at least one heterologous moiety.

8. Trimeric polypeptide complex according to claim 1, wherein each one of the three monomer polypeptides is covalently linked to at least one heterologous moiety.

9. A polynucleotide encoding a monomer polypeptide, said monomer polypeptide comprising a collagen XVIII trimerizing structural element (XVIIITSE) and being covalently linked to at least one heterologous moiety, wherein said heterologous moiety is a polypeptide and said heterologous moiety is positioned C-terminally to said monomer polypeptide.

10. A process for producing a trimeric polypeptide complex according to any one of claims 1 to 8, which comprises isolating said trimeric polypeptide complex from a culture comprising a host cell which carries and expresses a polynucleotide according to claim 9 which encodes at least one of the monomer polypeptides of said trimeric complex, and optionally subjecting the trimeric polypeptide complex to further processing.

11. A vector comprising a polynucleotide according to claim 10.

12. A pharmaceutical composition comprising a trimeric polypeptide complex according to any one of claims 1 to 8, a polynucleotide according to claim 9, a vector comprising said polynucleotide of claim 9, or a cell comprising said polynucleotide according to claim 9 or said vector comprising said polynucleotide according to claim 9, and at least a pharmaceutically acceptable carrier.

13. Use of a trimeric polypeptide complex according to any one of claims 1 to 8 as a vehicle for assembled antibody fragments to generate a chimeric artificial antibody having preselected pharmacokinetic and/or pharmacodynamic properties.
